# EUROPEAN PATENT APPLICATION

(11) **EP 3 782 993 A1**
(43) Date of publication of application: **24.02.2021**
(21) Application number: 19784682.7
(22) Date of filing: 12.04.2019
(51) Int. Cl.: C07D 401/14, C07D 213/73, A61K 31/4427

(54) **SELECTIVE JAK2 INHIBITOR AND APPLICATION THEREOF**

(30) Priority: 13.04.2018 CN 201810331657
(71) Applicant: East China University of Science and Technology, Shanghai 200237 (CN)
(72) Inventor: LI, Honglin, Shanghai 200237 (CN); XU, Yufang, Shanghai 200237 (CN); ZHAO, Zhenjiang, Shanghai 200237 (CN); ZHU, Lili, Shanghai 200237 (CN)
(74) Representative: reuteler & cie SA
(86) International application number: PCT/CN2019/082496
(87) International publication number: WO 2019/196937

(57) **Abstract**

Provided is a selective JAK2 inhibitor and application thereof. In particular, it relates to the compound of the following Formula (I), and the use of the compound in the treatment of JAK2-mediated related diseases and in the preparation of the medicament for treating JAK2-mediated related diseases.

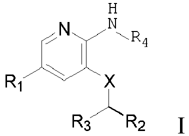

## Description

### Technical field

The present invention relates to the field of medicinal chemistry; and in particular, the present invention relates to a JAK2 inhibitor and uses thereof.

### Background

JAKs (Janus kinases) are non-receptor soluble protein tyrosine kinases in the cytoplasm. The JAKs family has four family members, namely JAK1, JAK2, JAK3 and TYK2 (Tyrosine kinase). JAK1, JAK2, and TYK2 are widely present in various tissues and cells *in vivo,* and JAK3 is mainly expressed in hematopoietic tissues. Among them, JAK2 is composed of 1132 amino acids, with a relative molecular mass of 13493, and its encoding gene is located in band 4 of region 2 of the short arm of chromosome 9. JAK2 consists of 7 JAK homology regions (JAK homology, JH) of different lengths from N-terminal to C-terminal. There are two homologous kinase domains JH1 and JH2 at the C-terminal; and five homologous regions JH3-JH7 at the N-terminal. JH1 is located at the carboxyl terminal, which is the catalytically active region of JAK2 and possess tyrosine kinase activity; JH2 exhibits no kinase activity, the function of which is to inhibit the effect of JH1; and JH3-JH7 can mediate the binding of JAK2 to cytokine receptors.

Signal transducers and activators of transcriptions (STAT) originated from the study of INF-γ signal transduction, which is a downstream substrate of JAK kinase. It transmits signals directly to the nucleus and exerts regulatory effects on the expression of specific genes. JAK-STAT is the Janus kinase-cell signal transduction and transcription activator pathway, and is a hot spot in the current research on cytokine. It plays an important role in a variety of physiological processes, such as cell growth, differentiation, immune function and hematopoiesis. JAK-STAT signal transduction usually includes four processes: ① cytokine binds to a corresponding ligand thereof, thereby inducing receptor dimerization; ② receptors and JAKs aggregate, so that neighboring JAKs phosphorylate each other and then be activated; ③ the JH1 domain of JAKs catalyzes the phosphorylation of tyrosine residues in the corresponding parts of STATs. At the same time, the SH2 functional region of STATs interacts with the phosphorylated tyrosine residues in the receptor to activate STATs; ④ STATs enter the nucleus in a homo or heterodimerization and multimerization forms and interact with other transcription factors to regulate gene transcription, thereby completing the whole process of cytokine-mediated signal transduction.

The lack of function of the JAK-STAT signaling pathway prevents cytokines from transmitting signals through the receptor, resulting in immunodeficiency. In many malignant solid tumors, such as colon cancer, head and neck cancer, lung cancer, breast cancer, and some blood diseases such as leukemia, lymphoma, multiple myeloma, persistent activation of JAK2 has been found. JAK kinase is a very important drug target. JAK inhibitors developed for this target are mainly used to screen therapeutic drugs for hematological diseases, tumors, rheumatoid arthritis and psoriasis. 31 kinase inhibitors have been approved by the FDA for clinical use so far. JAK kinase inhibitors tofacitinib and Jakafi (ruxolitinib) are used in the clinical treatment of rheumatoid arthritis and myelofibrosis, respectively.

JAK2 inhibitors reduce some inflammatory response cytokines by affecting the JAK2-STAT pathway, thereby affecting the regulation of the products and achieving clinical effects. The development of JAK2 inhibitors is not only of great significance to the treatment of myelofibrosis, but some JAK2 inhibitors will replace traditional drugs and open up new ways for the treatment of myeloproliferative tumors. The above-mentioned studies on JAK2 inhibitors for the treatment of immune inflammation and myeloproliferative cancer have achieved good results, but the safety needs to be further evaluated in clinical studies, and some inhibitors are not highly selective and will effect a variety of targets, thereby causing side effects. Therefore, it is very important for the research of highly selective and efficient targeted JAK2 inhibitors.

Summing up, the research and development of small molecule inhibitors targeting JAK2 kinase has important clinical significance and application prospects.

### Summary of the invention

The purpose of the present invention is to design and synthesize a JAK2 inhibitor with high efficiency, high selectivity, low toxicity and good safety.

In the first aspect of the present invention, a compound represented by formula I or a stereoisomer or optical isomer, or a pharmaceutically acceptable salt, prodrug or solvate thereof is provided, 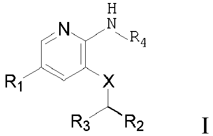 wherein
R₁ is selected from the following group: a hydrogen, halogen, C₁-C₁₀ alkyl, halogenated C₁-C₁₀ alkyl, C₁-C₁₀ alkoxy, halogenated C₁-C₁₀ alkoxy, substituted or unsubstituted phenyl, substituted or unsubstituted benzyl, substituted or unsubstituted 5-membered or 6-membered heterocyclic ring, substituted or unsubstituted 9-membered or 10-membered heterocyclic ring, -NR₅(R₆); the "substituted" means that the above group is replaced by oxo(=O) or one or more of the hydrogen atoms on the above group are replaced by a group selected from the following group: a C₁-C₄ alkyl (preferably methyl), cyano, -R₇-O-R₈, substituted or unsubstituted 4- to 6-membered ring containing one or two heteroatoms selected from nitrogen, oxygen or sulfur;
R₅ is selected from the following group: a hydrogen, C₁-C₄ alkyl, halogenated C₁-C₄ alkyl; R₆ is selected from the following group: C₁-C₁₀ alkyl, halogenated C₁-C₁₀ alkyl, substituted or unsubstituted phenyl, substituted or unsubstituted benzyl, substituted or unsubstituted 5-membered or 6-membered heterocycle, a substituted or unsubstituted 9-membered or 10-membered heterocycle; wherein the "substituted" means that one or more hydrogen atoms of the above group are replaced by a group selected from the following group: C₁-C₄ alkyl, cyano, halogen;
R₇ is an unsubstituted or substituted or unsubstituted C₁-C₆ alkylene group; R₈ is selected from the following group: a C₁-C₆ alkyl, halogenated C₁-C₆ alkyl, substituted or unsubstituted 4- to 6-membered cyclic group containing one or two heteroatoms selected from nitrogen, oxygen or sulfur, substituted or unsubstituted 4- to 6-membered cyclic group-C₁-C₄ alkyl containing one or two heteroatoms selected from nitrogen, oxygen or sulfur;
R₂ is selected from the following group: a substituted or unsubstituted phenyl, substituted or unsubstituted pyridyl, substituted or unsubstituted pyrimidine or substituted or unsubstituted C₅-C₇ cycloalkyl; and the "substituted" means that one or more hydrogen atoms of the above group is replaced by a group selected from the following group: a hydrogen, halogen, C₁-C₃ alkyl, halogenated C₁-C₃ alkyl, C₁-C₃ alkoxy, halogenated C₁-C₃ alkoxy;
R₃ is selected from the following group: a hydrogen, halogen, C₁-C₁₀ alkyl, halogenated C₁-C₁₀ alkyl, hydroxy, C₁-C₁₀ alkoxy;
R₄ is selected from the group consisting of a hydrogen, C₁-C₁₀ alkyl, C₁-C₁₀ acyl;
X is selected from the following group: CH₂, O, NH, S, SO, SO₂;

Unless otherwise specified, the "substituted" as mentioned above means that one or more hydrogens of a group (e.g., a C₁-C₆ alkylene, 4- to 6-membered ring groups containing one or two heteroatoms selected from nitrogen, oxygen or sulfur, 4- to 6-membered cyclic group-C1-C4 alkyl containing one or two heteroatom selected from nitrogen, oxygen or sulfur) are replaced by a group selected from the following group: a C₁-C₄ alkyl, C₁-C₄ haloalkyl, cyano and halogen.

In another preferred embodiment, the compound is represented by formula II: 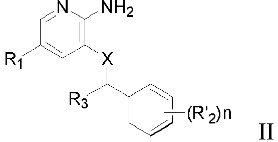 wherein
the definitions on R₁ and R₃ are the same as above;
R₂' is selected from the group consisting of a hydrogen, halogen, C₁-C₃ alkyl, halogenated C₁-C₃ alkyl, C₁-C₃ alkoxy, and halogenated C₁-C₃ alkoxy;
X is selected from the following group: O, NH, S;
n is 1, 2, 3, or 4.

In another preferred embodiment, X is selected from the following group: O, S.

In another preferred embodiment, R₃ is methyl.

In another preferred embodiment, the 5-membered or 6-membered heterocyclic ring or the 9-membered or 10-membered heterocyclic ring contains at least one nitrogen heteroatom.

In another preferred embodiment, the 5-membered or 6-membered heterocyclic ring or the 9-membered or 10-membered heterocyclic ring contains 1, 2 or 3 heteroatoms selected from nitrogen, oxygen or sulfur.

In another preferred example, the 5-membered or 6-membered heterocyclic ring or the 9-membered or 10-membered heterocyclic ring contains only 1, 2 or 3 heteroatoms, and the heteroatom is nitrogen.

In another preferred embodiment, the 9-membered or 10-membered heterocyclic ring contains 1, 2 or 3 nitrogen heteroatoms.

In another preferred embodiment, the 5-membered or 6-membered heterocyclic ring contains only 1, 2 or 3 heteroatoms, and the heteroatom is nitrogen.

In another preferred embodiment, the 9-membered or 10-membered heterocyclic ring is a condensed ring.

In another preferred embodiment, the 5-membered or 6-membered heterocyclic ring is selected from the following group: pyrrole, pyrazole, pyridine.

In another preferred embodiment, the 9-membered or 10-membered heterocyclic ring is selected from the following group:

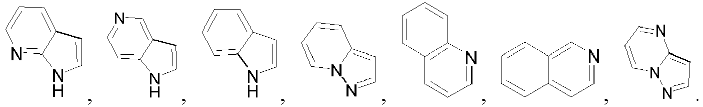

In another preferred embodiment, R₁ is selected from the following group: a substituted or unsubstituted 5-membered or 6-membered heterocyclic ring; wherein the "substituted" means that one or more hydrogen atoms of the above group are replaced by a group selected from the following group: a C₁-C₄ alkyl (preferably methyl), cyano, -R₇-O-R₈, saturated 4- to 6- membered ring containing one or two heteroatoms selected from nitrogen, oxygen or sulfur; wherein R₇ is a C₁-C₆ alkylene (preferably, a C₁-C₆ linear alkylene); and R₈ is selected from the group consisting of a C₁-C₆ alkyl and halogenated C₁-C₆ alkyl.

In another preferred embodiment R₁ is selected from the following group:

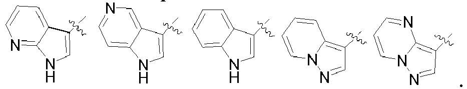

In another preferred embodiment, the 4- to 6- membered ring containing one or two heteroatoms selected from nitrogen, oxygen or sulfur is 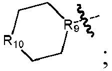 wherein, R₁₀ is selected from: NH₂, oxygen, and sulfur; and R₉ is selected from: N, CH.

In another preferred embodiment, the 4- to 6- membered ring containing one or two heteroatoms selected from nitrogen, oxygen or sulfur is selected from the group consisting of piperidine, piperazine, morpholine, oxetane and tetrahydrofuran.

In another preferred embodiment, 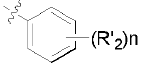 is a group selected from the following group: 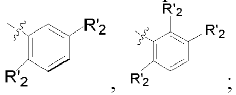 wherein the definitions on R₂' are the same as above.

In another preferred embodiment, the compound is selected from the following group:

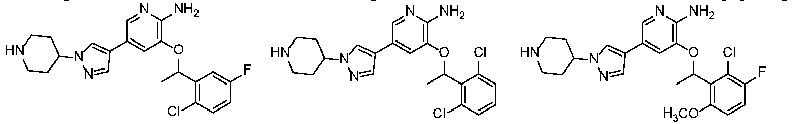

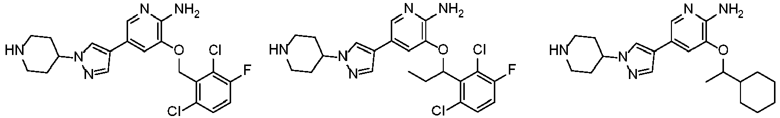

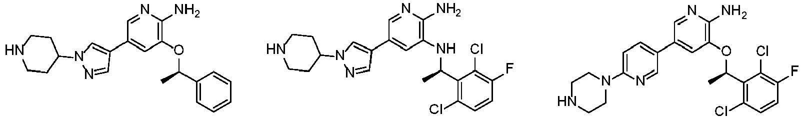

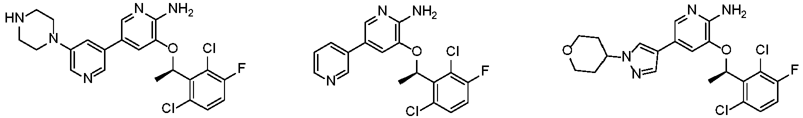

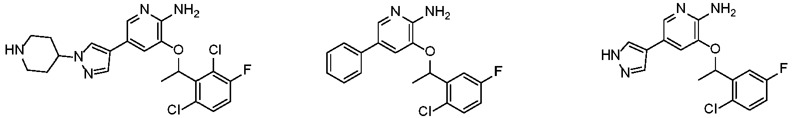

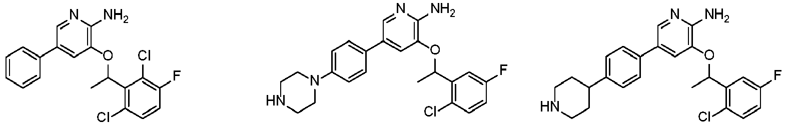

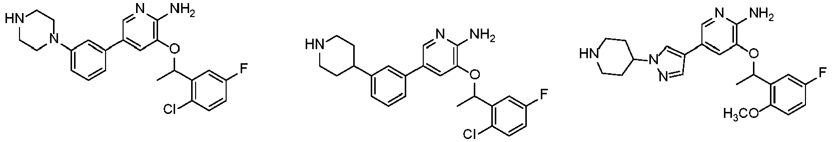

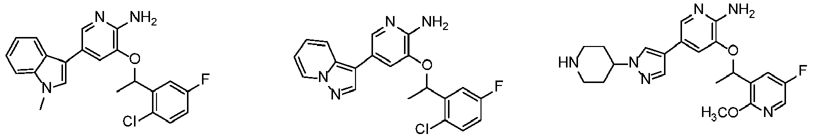

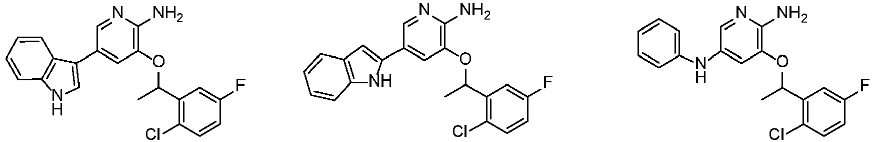

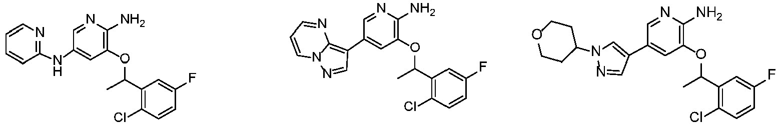

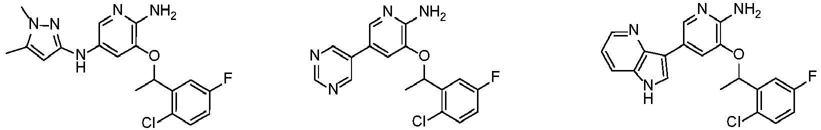

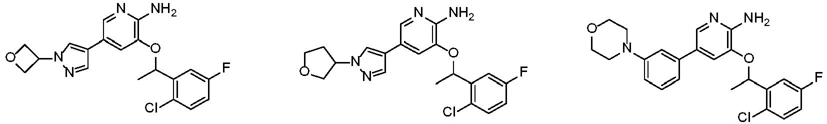

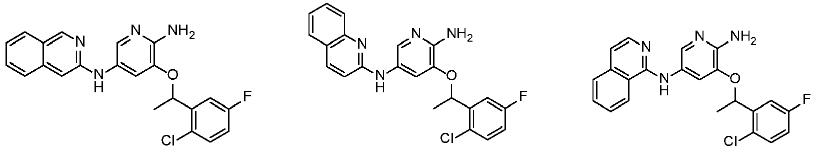

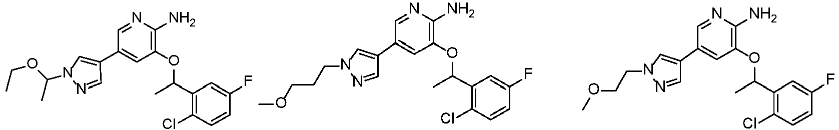

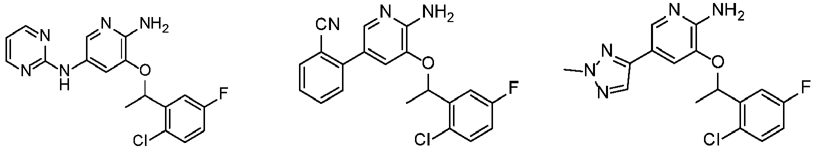

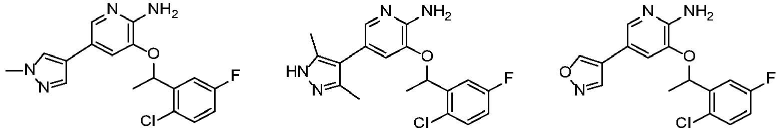

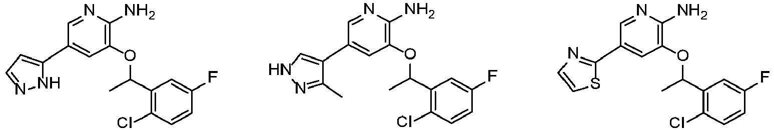

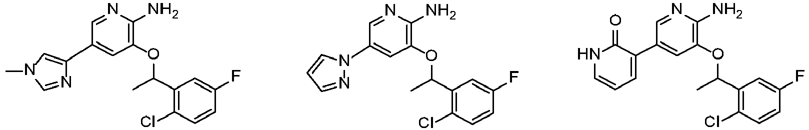

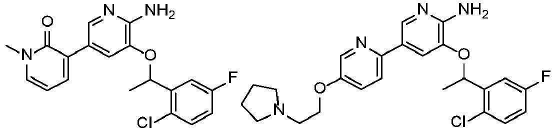

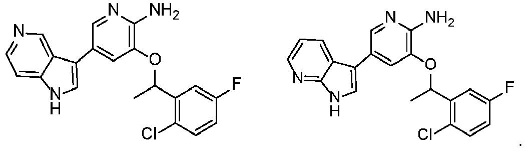

In the second aspect of the present invention, a pharmaceutical composition is provided, comprising the compound of the first aspect or a stereoisomer or optical isomer, or a pharmaceutically acceptable salt, prodrug or solvate thereof, and a pharmaceutically acceptable carrier or excipient.

In the third aspect of the present invention, the use of the compound of the first aspect or a stereoisomer or optical isomer, or pharmaceutically acceptable salt, prodrug or solvate thereof is provided, for preparing a medicament for preventing or treating JAK2-mediated diseases; and/or for preparing JAK2 inhibitors.

In another preferred embodiment, the JAK2-mediated disease is myelodysplastic syndrome (MDS), eosinophilia, tumor, inflammatory disease, or infection caused by bacteria, viruses or fungi.

In another preferred embodiment, the tumor is selected from the group consisting of: myeloproliferative carcinoma (MPN), melanoma, lung cancer (preferably non-small cell lung cancer), kidney cancer, ovarian cancer, prostate cancer, breast cancer, colon cancer, bone Cancer, pancreatic cancer, skin cancer, head and neck cancer, uterine cancer, rectal cancer, anal cancer, stomach cancer, testicular cancer, fallopian tube cancer, endometrial cancer, cervical cancer, vagina cancer, vulvar cancer, Hodgkin's disease, non Hodgkin's lymphoma, esophageal cancer, small bowel cancer, endocrine system cancer, thyroid cancer, parathyroid cancer, adrenal cancer, soft tissue sarcoma, urethral cancer, penile cancer, acute myeloid leukemia, chronic myelogenous leukemia, acute lymphoblastic leukemia , chronic lymphocytic leukemia, pediatric solid tumors, lymphocytic lymphoma, bladder cancer, kidney or ureter cancer, renal pelvis cancer, central nervous system (CNS) tumors, primary CNS lymphomas, tumor angiogenesis, spinal axons, glioma of brain stem, pituitary adenoma, Kaposi's sarcoma, epidermoid carcinoma, squamous cell carcinoma, T cell lymphoma.

In another preferred embodiment, the inflammatory disease is selected from the group consisting of rheumatoid arthritis, ankylosing spondylitis, autoimmune hemolytic anemia, arthritis, myasthenia gravis, systemic lupus erythematosus, pernicious anemia, polymyositis.

In another preferred embodiment, the virus is selected from the group consisting of hepatitis virus (type A, B and C), sporangia virus, influenza virus, adenovirus, coronavirus, measles virus, dengue fever virus, polio virus, rabies virus.

In another preferred embodiment, the bacteria are selected from the group consisting of chlamydia, rickettsiae, mycobacteria, staphylococci, pneumococcus, vibrio cholerae, and clostridium tetani.

In another preferred embodiment, the fungus is selected from the group consisting of Candida, Aspergillus, and Saccharomyces dermatitis.

In another preferred embodiment, the MPN classification includes chronic myeloid leukemia, polycythemia vera, essential thrombocythemia, primary myelofibrosis, chronic neutrophil leukemia, chronic eosinophilic leukemia, mastocytosis symptoms and unclassified MPN-U.

In another preferred embodiment, MDS/MPN includes chronic myelomonocytic leukemia, juvenile myelomonocytic leukemia, atypical chronic myelogenous leukemia and unclassified MDS/MPN-U (such as Refractory anaemia with ring sideroblasts associated with marked thrombocytosis (RARS-T)).

In the fourth aspect of the present invention, a JAK2 inhibitor is provided, comprising the compound of the first aspect of the present invention or a stereoisomer or optical isomer, or a pharmaceutically acceptable salt, prodrug or solvate thereof or the pharmaceutical composition of the second aspect.

The present invention provides a treatment method, comprising the steps of: administering the compound of the first aspect of the present invention or a stereoisomer or optical isomer, or a pharmaceutically acceptable salt, prodrug or solvate thereof, to a subject in need thereof.

In another preferred embodiment, the subject in need thereof suffers from a JAK2-mediated disease.

It should be understood that within the scope of the present invention, the above-mentioned technical features of the present invention and the technical features specifically described in the following (such as the embodiments) can be combined with each other to form a new or preferred technical solution, and it is not necessary to repeat them one-by-one.

### Description of drawings

Figure 1 is the chiral resolution chromatogram of MXY-193 (220nm).
Figure 2 is the chiral resolution chromatogram of MXY-193 (254nm).

### Modes for carrying out the invention

Through extensive and in-depth research, the inventors have unexpectedly discovered a series of aminopyridine compounds, which can selectively inhibit JAK2 kinase, and thus become small molecule leading drugs for studying JAK2 inhibitors, thereby providing a basis for developing immuno-inflammatory and anti-tumor drugs. Based on the above findings, the present invention has been completed.

Unless otherwise defined, all technical and scientific terms used herein have the same meaning as commonly understood by a skilled person in the art to which the disclosed invention belongs. To facilitate the understanding of the present invention, the relevant terms involved in the present invention are defined as follows, but the scope of the present invention is not limited to these specific definitions.

### Terms

As used herein, "JAK2" refers to Janus kinase 2, which is a cytoplasmic non-receptor soluble protein tyrosine kinase. JAK-STAT is the Janus kinase-cell signal transduction and transcription activator pathway, and is a hot spot in the current research on cytokine.

As used herein, "C₁-C₁₀ alkyl" refers to a saturated branched or straight chain alkyl having 1-10 carbon atoms in length, and a preferred alkyl group is an alkyl of 1-3 carbon atoms. Examples of alkyl include, but are not limited to, methyl, ethyl, n-propyl, isopropyl, n-butyl, isobutyl, heptyl and the like.

As used herein, "C₅-C₇ cycloalkyl" refers to a saturated cycloalkyl with 5-7 carbon atoms in length, such as cyclopentyl, cyclohexyl and the like.

As used herein, "C₁-C₆ alkylene" includes linear or branched alkylene, and preferred alkylene is an alkylene containing 1 to 3 carbon atoms. Examples of alkylene include, but are not limited to, CH₂, (CH₂)₂, (CH₂)₂, CH(CH₃), CH(CH₃)CH₂, and the like.

As used herein, "alkoxy" refers to an oxy substituted by an alkyl. A preferred alkoxy is an alkoxy with 1-10 carbon atoms, more preferably 1-4 carbon atoms in length. Examples of alkoxy groups include, but are not limited to, methoxy, ethoxy, propoxy and the like. In specific embodiment, the alkoxy may be a substituted alkoxy, for example, a halogen-substituted alkoxy. In a specific embodiment, a halogen-substituted C₁-C₃ alkoxy is preferred.

As used herein, "heterocyclic group" or "heterocyclic ring" includes, but is not limited to, 5-membered or 6-membered heterocyclic groups containing 1-3 heteroatoms selected from O, S or N, including but not limited to furyl, thienyl, pyrrolyl, pyrrolidinyl, pyrazolyl, imidazolyl, triazolyl, oxazolyl, pyranyl, pyridyl, pyrimidinyl, pyrazinyl, piperidinyl, morpholinyl, isoindolyl, and the like.

As used herein, "halogen" refers to fluorine, chlorine, bromine and iodine. In a preferred embodiment, halogen is chlorine or fluorine.

As used herein, "halo" refers to fluoro, chloro, bromo and iodo.

### Active ingredients

As used herein, "the compound of the present invention" refers to the compound represented by formula (I), and also includes various crystal forms, pharmaceutically acceptable salts, hydrates or solvates of the compound of formula (I).

As used herein, "pharmaceutically acceptable salt" refers to a salt suitable for use as a medicine, which is formed by a compound of the present invention and an acid or base. Pharmaceutically acceptable salts include inorganic salts and organic salts. A preferred class of salt is a salt formed from the compounds of this invention with acids. The acid suitable for forming a salt includes but not limited to: an inorganic acid, such as hydrochloric acid, hydrobromic acid, hydrofluoric acid, sulfuric acid, nitric acid, phosphoric acid, an organic acid, such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, and benzenesulfonic acid; and an acidic amino acid, such as aspartic acid and glutamic acid.

Unless otherwise specified, the structural formula described in the present invention is intended to include all isomeric forms (such as enantiomers, diastereomers and geometric isomers (or conformational isomers)): for example, R and S configurations containing an asymmetry center, (Z) and (E) isomers of the double bond, etc. Therefore, a single stereochemical isomer of the compound of the present invention or a mixture of enantiomers, diastereomers or geometric isomers (or conformational isomers) thereof will fall within to the scope of the present invention.

"Tautomers" means that structural isomers with different energies can exceed the low energy barrier to convert into each other. For example, proton tautomers (i.e., proton transfer) include interconversion through proton transfer, such as 1H-indazole and 2H-indazole. Valence tautomers include interconversion through some bond-forming electron recombination.

"Solvate" refers to a complex with a specific ratio formed by coordination of the compound of the present invention with solvent molecules.

"Hydrate" refers to a complex formed by coordination of the compound of the present invention with water.

### Pharmaceutical composition and method of administration

Since the compound of the present invention exhibits excellent JAK kinase inhibitory activity, the compound of the present invention and various crystal forms, pharmaceutically acceptable inorganic or organic salts, hydrates or solvates thereof, and a pharmaceutical composition containing the compound of the present invention as the main active ingredient can be used to prevent and/or treat (stabilize, reduce or cure) JAK kinase related diseases.

The pharmaceutical composition of the present invention comprises a safe and effective amount of the compound of the present invention and a pharmaceutically acceptable excipient or carrier. The "safe and effective amount" means that the amount of the compound is sufficient to significantly improve the condition without causing serious side effects. Generally, the pharmaceutical composition contains 1-2000 mg of the compound of the present invention per agent, and more preferably, 10-200 mg of the compound of the present invention per agent. Preferably, the "one dose" is one capsule or tablet.

"Pharmaceutically acceptable carrier" refers to: one or more compatible solid or liquid fillers or gel substances, which are suitable for human use, and must have sufficient purity and sufficiently low toxicity. As used herein, "compatibility" means that each component in the composition can be blended with the compound of the present invention without significantly reducing the efficacy of the compound. Examples of pharmaceutically acceptable carriers include cellulose and a derivative thereof (such as sodium carboxymethyl cellulose, sodium ethyl cellulose, cellulose acetate, etc.), gelatin, talc, and solid lubricants (such as stearic acid, Magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween®), wetting agents (such as sodium lauryl sulfate), coloring agents, flavoring agents, stabilizers, antioxidants, preservatives, pyrogen-free water, etc.

The method for administering the compound or pharmaceutical composition of the present invention is not particularly limited, and representative administration methods include (but are not limited to): oral, parenteral (intravenous, intramuscular, or subcutaneous) administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with the following ingredients: (a) fillers or compatibilizers, for example, Starch, lactose, sucrose, glucose, mannitol and silicic acid; (b) binders such as hydroxymethyl cellulose, alginate, gelatin, polyvinylpyrrolidone, sucrose and gum arabic; (c) humectants, For example, glycerin; (d) disintegrating agents, such as agar, calcium carbonate, potato starch or tapioca starch, alginic acid, certain complex silicates, and sodium carbonate; (e) slow solvents, such as paraffin; (f) Absorption accelerators, such as quaternary amine compounds; (g) wetting agents, such as cetyl alcohol and glyceryl monostearate; (h) adsorbents, such as kaolin; and (i) lubricants, such as talc, hard Calcium fatty acid, magnesium stearate, solid polyethylene glycol, sodium lauryl sulfate, or mixtures thereof. In capsules, tablets and pills, the dosage form may also contain buffering agents.

Solid dosage forms, such as tablets, sugar pills, capsules, pills and granules can be prepared with coatings and shell materials, such as enteric coatings and other materials known in the art. They may contain opacifying agents, and the active compound or the release of the compound in such a composition may be released in a certain part of the digestive tract in a delayed manner. Examples of embedding components that can be used are polymeric substances and waxes. If necessary, the active compound can also be formed into microcapsules with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures thereof.

In addition to these inert diluents, the composition may also contain adjuvants, such as wetting agents, emulsifying and suspending agents, sweetening agents, flavoring agents and perfumes.

In addition to the active compound, the suspension may contain suspending agents, for example, ethoxylated isostearyl alcohol, polyoxyethylene sorbitol and sorbitan esters, microcrystalline cellulose, aluminum methoxide and agar, or mixtures thereof, and the like.

The composition for parenteral injection may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The compound of the present invention can be administered alone or in combination with other pharmaceutically acceptable compounds.

When administered in combination, the pharmaceutical composition also includes one or more (2, 3, 4, or more) other pharmaceutically acceptable compounds. One or more of the other pharmaceutically acceptable compounds can be administered simultaneously, separately or sequentially with the compounds of the invention.

When using the pharmaceutical composition, a safe and effective amount of the compound of the present invention is applied to a mammal (such as a human) in need of the treatment, wherein the administered dosage is a pharmaceutically effective dosage. For a human with a body weight of 60 kg, the daily dosage is usually 1 to 2000 mg, preferably 20 to 500 mg. When determining a specific dosage, factors such as the route of administration, the patient's health status and the like should also be considered, which are within the skill of a skilled physician.

### Preparation method

The compounds of the present invention can be prepared according to conventional routes or methods, and can also be obtained according to the methods or routes described herein, for example, Route 1 or Route 2.

Synthetic route 1:

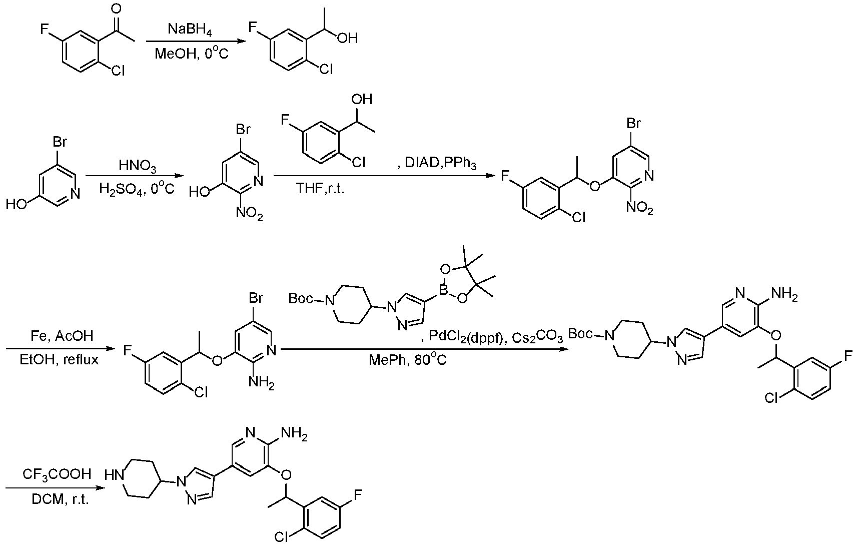

Synthetic route 2:

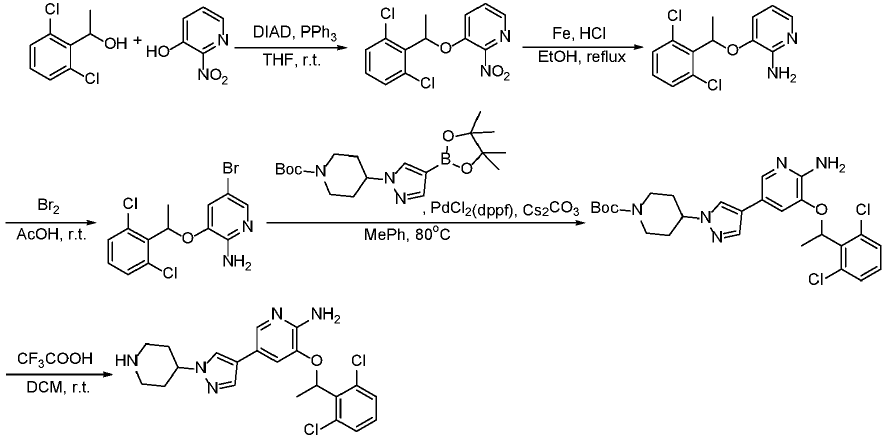

### Main advantages of the present invention:

1. The compound of the present invention has a novel structure and excellent JAK kinase inhibitor effects;
2. The compound of the present invention has better selectivity on inhibiting JAK1.

The present invention will be further described below in combination with specific embodiments. It should be understood that these embodiments are only used to illustrate the present invention and not to limit the scope of the present invention. The experimental methods, specific conditions of which are not indicated in the following examples, usually follow the conventional conditions or the conditions recommended by the manufacturer. Unless otherwise specified, percentages and parts are weight percentages and parts by weight.

### Example 1 Preparation of compound WWQ-131

### 1) Synthesis of 1-(2-chloro-5-fluorophenyl)ethanol

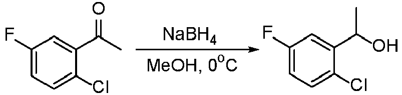

The raw material (1 g, 5.8 mmol) was placed into a 50 mL reaction flask, dissolved in anhydrous methanol under argon, and stirred in an ice bath for 30 minutes. Afterwards, NaBH4 (331 mg, 8.7 mmol) was slowly add, gradually increased to room temperature, and stirred at room temperature. The reaction was monitored by TLC, and after 6 hours, the reaction was complete. The reaction was quenched by adding water under an ice bath, and the methanol was removed *in vacuo.* DCM and water were added for extraction for three times, and the organic phase was extracted for three times with saturated brine. The organic phases were collected, dried over sodium sulfate, and suction-filtered. The organic phases were dried *in vacuo* to obtain a colorless oily liquid (960 mg, a yield of 95%). ¹H NMR (400 MHz, CDCl₃): δ 7.25 (dd, *J*₁ = 2.4 Hz, *J*₂ = 6.8 Hz, 1H), 7.20 (dd, *J*₁ = 4.0 Hz, *J*₂ = 5.2 Hz, 1H), 6.83 (td, *J*₁ = 2.8 Hz, *J*₂ = 8.4 Hz, 1H), 5.15 (q, *J* = 6.4Hz, 1H), 2.06 (s, 1H), 1.39 (d, *J =* 6.4 Hz, 3H). GC-MS: m/z: 174.1.

### 2) 3-Hydroxy-5-bromo-2-nitropyridine

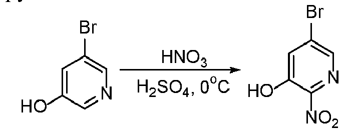

3-hydroxy-5-bromo-pyridine (25 g, 0.145 mol) was placed into a 250 mL reaction flask, and 50 mL of concentrated sulfuric acid was added to dissolve it and stirred in an ice bath for 30 minutes. Concentrated nitric acid (19.5 g, 0.201 mol) was slowly added dropwise, gradually warmed to room temperature, and stirred at room temperature. The reaction was monitored by TLC. After 6 hours, the reaction was completed. The reaction solution was slowly added dropwise into ice water. White solids precipitated and were suction-filtered. The filter cake was washed with water for 3 times, and dried in an oven, so as to give white solids (21.5 g, yield of 68.25%). ¹H NMR (400 MHz, CDCl₃): δ 10.28 (s, 1H), 8.24 (d, *J* = 2.0 Hz, 1H). GC-MS: m/z: 218.1.

### 3) 3-[1-(2-chloro-5-fluorophenyl)ethoxy]-5-bromo-2-nitropyridine

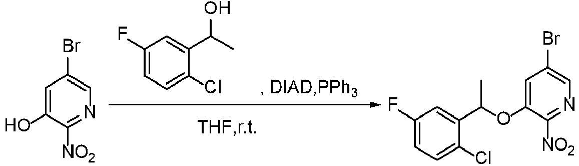

1-(2-chloro-5-fluorophenyl)ethanol (2.20 g, 12.6 mmol), 3-hydroxy-5-bromo-2-nitropyridine (2.75 g, 12.6 mmol), triphenylphosphine (3.97 g, 15.1 mmol) were weighted into a 50 mL two-necked flask and THF was added to dissolve them. Under argon, DIAD (3.06 g, 15.1 mmol) was slowly added dropwise with stirring in an ice bath, and upon addition, warmed up to room temperature. The reaction was monitored by TLC. After 4 hours, the reaction was complete. Silica gel column chromatography (PE to PE: EA=100: 1) was performed to obtain 4.232 g of white solids with a yield of 89.5%. ¹H NMR (400 MHz, CDCl₃): δ 8.11 (d, *J*= 2.0 Hz, 1H), 7.40 (dd, *J*₁ = 4.8 Hz, *J*₂ = 8.4 Hz, 1H), 7.38 (d, *J=* 1.6 Hz, 1H), 7.24 (dd, *J*₁ = 2.8 Hz, *J*₂ = 8.8 Hz, 1H), 7.03-6.99 (m, 1H), 5.78 (q, *J= 6.4* Hz, 1H), 1.69 (d, *J* = 6.0 Hz, 3H). GC-MS: m/z = 375.9.

### 4) 3-[1-(2-chloro-5-fluorophenyl)ethoxy]-5-bromo-2-aminopyridine

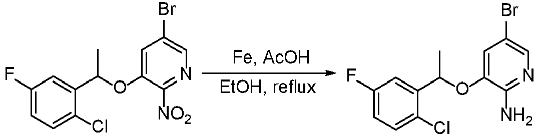

3-[1-(2-chloro-5-fluorophenyl)ethoxy]-5-bromo-2-nitropyridine (4.232 g, 11.3 mmol) and iron powder (1.58 g, 28.2 mmol) were weighted into a 100 mL reaction flask, and ethanol and acetic acid (1:1) were added to dissolve them, and refluxed at 78° C. The reaction was monitored by TLC. After 3 hours, the reaction was complete. The reaction liquid was dried *in vacuo.* The pH was adjusted to alkaline by adding NaOH. The solids were filtered and the filtrate was extracted fot three times with DCM/H₂O. The organic phase was extracted for three times with saturated brine, dried over anhydrous sodium sulfate, and suction-filtered. The filtrate was dried *in vacuo,* and silica gel column chromatography (DCM) was performed to obtain 3.735 g of milky white solids with a yield of 96.25%. ¹H NMR (400 MHz, CDCl₃): δ 7.68 (d, *J* = 1.6 Hz, 1H), 7.38 (dd, *J*₁ = 4.8 Hz, *J*₂ = 8.8 Hz, 1H), 7.09 (dd, *J*₁ = 3.2 Hz, *J*₂ = 9.2 Hz, 1H), 7.00-6.95 (m, 1H), 6.70 (d, *J=* 1.6 Hz, 1H), 5.59 (q, *J* = 6.4 Hz, 1H), 4.86 (s, 1H), 1.66 (d, *J=* 6.4 Hz, 3H). GC-MS: m/z = 345.9.

### 5) Tert-butyl 4-{3-[1-(2-chloro-5-fluorophenyl)ethoxy]-2-aminopyridine}pyrazol-1-carboxylate piperidine

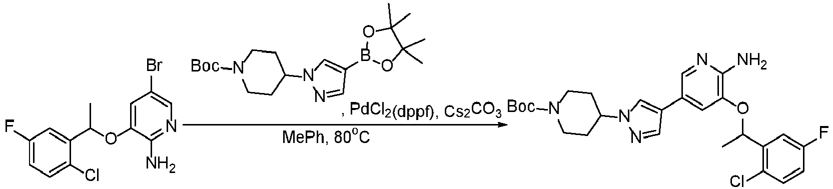

3-[1-(2-chloro-5-fluorophenyl)ethoxy]-5-bromo-2-aminopyridine (3.735 g, 10.8 mmol), tert-butyl 4-[4-(4,4,5,5-tetramethyl-1,3,2-dioxaborolan-2-yl)-1H-pyrazol-1-yl)piperidine-1-carboxylate (4.915 g, 13.0 mmol) and cesium carbonate (12.382 g, 38.0 mmol) were added to a 100 mL three-necked flask, and toluene and water (10:1) were added to dissolve them. Argon was purged to deoxygenate for 20 minutes, and Pd catalyst was added to deoxygenate for another 10 minutes. Under argon, the reaction system was refluxed and stirred at 80° C. The reaction was monitored by TLC. After 12 hours, the reaction was completed and dried *in vacuo.* Silica gel column chromatography (DCM: MeOH = 100: 1) was performed to obtain 4.76 g of yellow oily liquid with a yield of 85%. ¹H NMR (400 MHz, CDCl₃): δ 7.75 (d, *J=* 1.6 Hz, 1H), 7.53 (s, 1H), 7.46 (s, 1H), 7.37 (dd, *J*₁ = 5.6 Hz, *J*₁ = 8.8 Hz, 1H), 7.15 (dd, *J*₁ = 3.2 Hz, *J*₂ = 9.2 Hz, 1H), 6.97-6.92 (m, 1H), 6.75 (d, *J* = 1.6 Hz, 1H), 5.70 (q, *J* = 6.0 Hz, 1H), 4.84 (s, 2H), 4.27-4.21 (m, 1H), 2.89 (t, *J=* 12.0 Hz, 2H), 2.12 (dd, *J*₁ = 2.0 Hz, *J*₂ = 12.4 Hz, 2H), 1.96-1.89 (m, 4H), 1.68 (d, *J* = 6.4 Hz, 3H), 1.48 (s, 9H).

### 6) 4-{3-[1-(2-chloro-5-fluorophenyl)ethoxy]-2-aminopyridine}pyrazol-1-piperidine WWQ-131

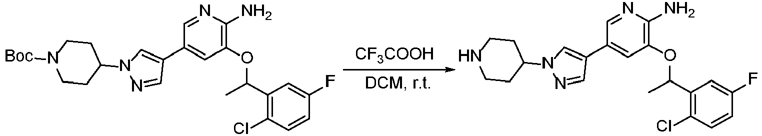

Tert-butyl 4-{3-[1 -(2-chloro-5-fluorophenyl)ethoxy] -2-aminopyridine}pyrazole-1-carboxylate piperidine (4.76 g, 9 mmol) was weighted, and DCM was added to dissolve it. Trifluoroacetic acid (12 mL) was added dropwise in an ice bath, and upon addition, warmed up to room temperature. The reaction was monitored by TLC. After 12 hours, the reaction was complete. The reaction system was neutralized to alkaline with saturated NaHCO₃, extracted with DCM/H₂O for three times. The organic phase was extracted for three times with saturated brine, dried over anhydrous sodium sulfate, and suction-filtered. The filtrate was dried *in vacuo.* Dichloromethane and petroleum ether were used for recrystallization, and 2.20 g of white solids were obtained through suction-filtration with a yield of 57.44%. ¹H NMR (400 MHz, CDCl₃): δ 7.78 (s, 1H), 7.54 (s, 1H), 7.48 (s, 1H), 7.37 (dd, *J*₁ = 4.8 Hz, *J*₂ = 8.8 Hz, 1H), 7.15 (dd, *J*₁ = 3.2 Hz, *J*₂ = 9.2 Hz, 1H), 6.96-6.91 (m, 1H), 6.74 (d, *J=* 1.6 Hz, 1H), 5.70 (q, *J* = 6.0 Hz, 1H), 4.79 (s, 2H), 4.26-4.21 (m, 1H), 3.31 *(d, J=* 12.4 Hz, 2H), 3.05 (m, 2H), 2.87-2.80 (m, 2H), 2.19 (d, *J* = 10.8 Hz, 2H), 2.03-1.94 (m, 2H), 1.67 (d, *J* = 6.4 Hz, 3H). ¹³C NMR (100 MHz, CDCl₃): δ 148.6, 141.8, 139.9, 135.9, 131.1, 126.2, 122.7, 119.9, 119.3, 116.6, 116.4, 115.8, 113.8, 113.5, 72.5, 58.8, 44.9, 32.8, 22.6. HRMS (ESI) (m/z): [M+H]⁺ calculated for C₂₁H₂₃ClFN₅O, 416.1609; found 416.1649. HPLC purity: 98.63%, retention time = 9.925 min.

### Example 2 Preparation of WWQ-133

### 1) 3-[1-(2,6-Dichlorophenyl)ethoxy]-2-nitropyridine

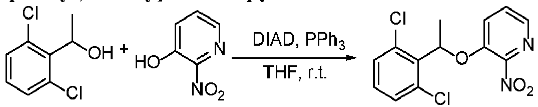

The raw materials 1-(2,6-dichlorophenyl)ethanol (1 g, 5.23 mmol), 3-hydroxy-2-nitropyridine (807 mg, 5.75 mmol), triphenylphosphine (1.647 g, 6.27 mmol) was weighted into a 50 mL two-necked flask and THF was added to dissolve them. Under argon, DIAD (1.236 mL, 6.27 mmol) was slowly added dropwise with stirring in an ice bath, and upon addition, warmed up to room temperature. The reaction was monitored by TLC. After 4 hours, the reaction was complete. Silica gel column chromatography (PE: EA = 50: 1) was performed to obtain 804 mg of white solids with a yield of 48.5%. H NMR (400 MHz, CDCl₃): δ 8.01 (dd, *J*₁ = 1.2 Hz, *J*₂ = 3.2 Hz, 1H), 7.36-7.33 (m, 1H), 7.32 (s, 1H), 7.30 (s, 1H), 7.23 (dd, *J=* 0.8, 7.6 Hz, 1H), 7.18 (t, *J=* 8.0 Hz, 1H), 6.13 (q, *J* = 6.4Hz, 1H), 1.85 (d, *J=* 6.8 Hz, 3H). GC-MS:m/z = 312.1.

### 2) 3-[1-(2,6-dichlorophenyl)ethoxy]-2-aminopyridine

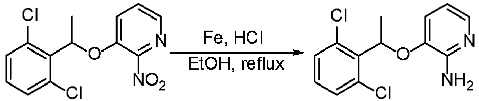

3-[1-(2,6-dichlorophenyl)ethoxy]-2-nitropyridine (804 mg, 2.56 mmol) and iron powder (360 mg, 6.41 mmol) were weighted into a 50 mL two-necked flask. EtOH was added to dissolve them and refluxed for 30 minutes at 90°C and then HCl was added. The reaction was monitored by TLC. The reaction was complete and dried *in vacuo.* NaOH was added to adjust the pH to alkaline. The reaction liquid was extracted for three times with DCM/H₂O. The organic phase was extracted for three times with saturated brine, dried over anhydrous sodium sulfate, and suction-filtered. The filtrate was dried *in vacuo* to give the product, which was subjected to silica gel column chromatography to obtain 650 mg of milky white solids with a yield of 89.9%. ¹H NMR (400 MHz, CDCl₃): δ 7.58 (d, *J* = 4.4 Hz, 1H), 7.29 (d, *J* = 8 Hz, 2H), 7.14 (t, *J* = 8 Hz, 1H), 6.73 (d, *J* = 7.6 Hz, 1H), 6.47 (t, *J* = 5.2 Hz, 1H), 6.40 (q, *J* = 6.4 Hz, 1H), 4.86 (s, 2H), 1.81 (d, *J*= 6.8 Hz, 3H). GC-MS: m/z = 282.0.

### 3) 3-[1-(2,6-dichlorophenyl)ethoxy]-5-bromo-2-aminopyridine

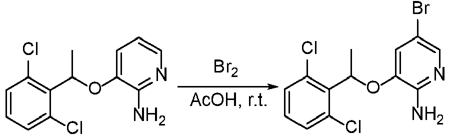

The product from the previous step (650 mg, 2.30 mmol) was placed into a 50 mL two-necked flask, and AcOH (10 mL) was added to dissolve it. Under argon, BR₂ (436 mg, 2.76 mmol) was added dropwise in an ice bath, and upon addition, warmed up to room temperature. The reaction was monitored by TLC. After 2 hours, the reaction was completed, and 480 mg of yellow solids were obtained with a yield of 57.9%. ¹H NMR (400 MHz, CDCl₃): δ 7.55 (d, *J* = 2.0 Hz, 1H), 7.34 (s, 1H), 7.32 (s, 1H), 7.18 (t, *J* = 8.0 Hz, 1H), 6.86 (d, *J* = 2.0 Hz, 1H), 6.02 (q, *J= 6.8* Hz, 1H), 5.53 (s, 2H), 1.83 (d, *J* = 6.8 Hz, 3H). GC-MS: m/z = 360.1.

### 4) Tert-butyl 4-{3-[1-(2,6-dichlorophenyl)ethoxy]-2-aminopyridine}pyrazol-1-carboxylate piperidine

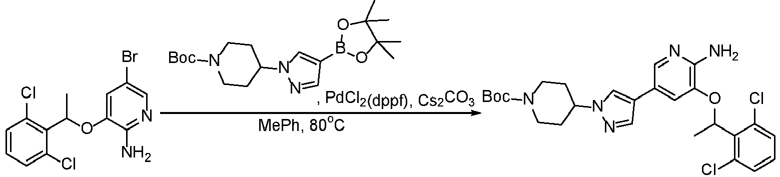

The brominated product (450 mg, 1.25 mmol), tert-butyl 4-[4-(4,4,5,5-tetramethyl-1,3,2-dioxaborolan-2-yl)-1H-pyrazol-1-yl]piperidine-1-carboxylate (565 mg, 1.5 mmol), cesium carbonate (1.425 g, 4.38 mmol) were added into a 50 mL three-necked flask, and 10 mL of toluene and 1 mL of water were added to dissolve them. Deoxygenation was performed for 20 minutes, then Pd catalyst was added, and deoxygenation was performed for another 10 minutes. Under argon protection, the reaction system was refluxed and stirred at 80°C. The reaction was monitored by TLC. After 12 hours, the reaction was complete, and dried *in vacuo.* Silica gel column chromatography (DCM: MeOH = 100: 1) was performed to give 260 mg of milky white solids with a yield of 39.1%. ¹H NMR (400 MHz, CDCl₃): δ 7.72 (d, *J* = 2.0 Hz, 1H), 7.56 (s, 1H), 7.49 (s, 1H), 7.32 (s, 1H), 7.30 (s, 1H), 7.15 (t, *J= 8.0* Hz, 1H), 6.90 (d, *J* = 1.6 Hz, 1H), 6.10 (q, *J* = 6.8 Hz, 1H), 4.88 (s, 2H), 4.28-4.21 (m, 1H), 2.90 (t, *J* = 11.2 Hz, 2H), 2.13 (d, *J* = 10.0 Hz, 2H), 2.04 (m, 2H), 1.93 (td, *J*₁ = 4.0 Hz, *J*₂ = 12.0 Hz, 2H), 1.85 (d, *J=* 6.8 Hz, 3H), 1.48 (s, 9H). LC-MS: m/z: 532.1 (M+H)⁺.

### 5) 4-{3-[1-(2,6-dichlorophenyl)ethoxy]-2-aminopyridine}pyrazol-1-piperidine WWQ-133

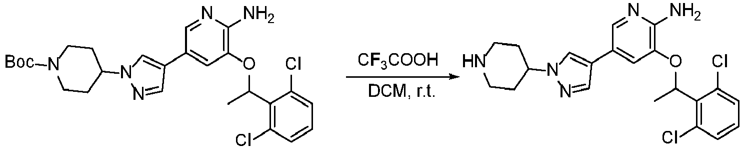

The product from the previous step (246 mg, 0.463 mmol) was weighted, and DCM was added to dissolve it. Trifluoroacetic acid (2 mL) was added dropwise in an ice bath, and upon addition, warmed up to room temperature. The reaction was monitored by TLC. After 3 hours, the reaction was complete. The reaction was neutralized to alkaline with saturated NaHCO₃, and extracted for three times with DCM/H₂O. The organic phase was extracted for three times with saturated brine, dried over anhydrous sodium sulfate, and suction-filtered. The filtrate was dried *in vacuo.* Silica gel column chromatography (DCM: MeOH = 50: 1) was performed to obtain 91 mg of white solids with a yield of 45.5%. ¹H NMR (400 MHz, CDCl₃): δ 7.48 (d, *J* = 2.0 Hz, 1H), 7.57 (s, 1H), 7.51 (s, 1H), 7.32 (s, 1H), 7.30 (s, 1H), 7.14 (t, *J* = 8.0 Hz, 1H), 6.90 (d, *J* = 1.6 Hz, 1H), 6.10 (q, *J= 6.8* Hz, 1H), 4.79 (s, 2H), 4.25-4.17 (m, 1H), 3.27 (d, *J* = 12.8 Hz, 2H), 2.79 (td, *J*₁ = 2.4 Hz, *J*₂ = 12.4 Hz, 2H), 2.19-2.12 (m, 2H), 1.96-1.89 (m, 2H), 1.86 (d, *J* = 6.4 Hz, 3H). ¹³C NMR (100 MHz, CDCl₃): δ 148.96, 140.0, 135.7, 135.3, 135.0, 134.5, 129.6, 122.5, 120.1, 119.2, 115.1, 72.3, 59.4, 45.4, 33.5, 19.0. HRMS (ESI) (m/z): [M+H]⁺ calculated for C₂₁H₂₃Cl₂N₅O, 432.1373; found 432.1361. HPLC purity: 97.82%, retention time = 9.935 min.

### Example 3 Preparation of WWQ-153 (WWQ series of compounds were synthesized according to the method similar to the example)

### 4-{3-[1-(2,6-dichloro-3-fluorophenyl)methoxy]-2-aminopyridine}pyrazol-1-piperidine

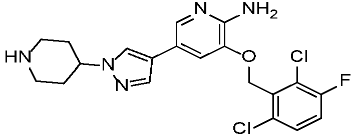

43 mg of white solids were obtained with a yield of 45.1%, melting point: 195.0-196.5 °C. ¹H NMR (400 MHz, CDCl₃): δ 7.87 (d, *J* = 2.0 Hz, 1H), 7.67 (s, 1H), 7.60 (s, 1H), 7.52 (dd, *J*₁ = 2.8 Hz, *J*₂ = 6.4 Hz, 1H), 7.39 (dd, *J*₁ =2.4 Hz, *J*₂ = 5.6 Hz, 1H), 7.07 (d, *J* = 1.6 Hz, 1H), 5.15 (s, 2H), 4.69 (s, 2H), 4.28-4.21 (m, 1H), 3.26 (d, *J=* 12.8 Hz, 2H), 2.78 (td, *J*₁ = 2.0 Hz, *J*₂ = 12.4 Hz, 2H), 2.19 (dd, *J*₁ = 2.0 Hz, *J*₂ = 12.4 Hz, 2H), 1.98-1.88 (m, 2H), 1.83 (s, 1H). ¹³C NMR (100 MHz, CDCl₃): δ 153.4, 148.6, 140.8, 136.7, 135.8, 130.2, 129.8, 127.7, 126.4, 126.2, 122.9, 122.4, 119.7, 115.2, 63.5, 59.9, 45.7, 34.0. HRMS (ESI) (m/z): [M+H]⁺ calculated for C₂₀H₂₀Cl₂FN₅O, 436.1062; found 436.1100. HPLC purity: 97.76%, retention time = 10.879 min.

### Example 4 Preparation of WWQ-175

### 4-{3-[1-(2-chloro-3-fluoro-6-methoxyphenyl)ethoxy]-2-aminopyridine}pyrazol-1-piperidine

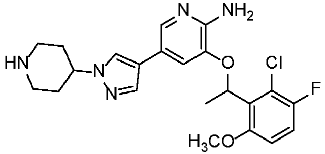

97 mg of white solids were obtained with a yield of 56.2%, melting point: 189.8-191.3 °C. ¹H NMR (400 MHz, CDCl₃): δ 7.73 (d, *J* = 1.6 Hz, 1H), 7.53 (s, 1H), 7.50 (s, 1H), 7.03 (t, *J* = 8.8 Hz, 1H), 6.96 (d, *J =* 2.0 Hz, 1H), 6.76 (dd, *J*₁ = 2.8 Hz, *J*₂ = 9.2 Hz, 1H), 6.06 (q, *J* = 6.4 Hz, 2H), 4.76 (s, 2H), 4.25-4.17 (m, 1H), 3.89 (s, 3H), 3.26 (d, *J* = 12.8 Hz, 2H), 2.78 (td, *J*₁ = 2.0 Hz, *J*₂ = 12.4 Hz, 2H), 2.17 (d, *J* = 12.4 Hz, 2H), 2.02 (s, 1H), 1.96-1.85 (m, 2H), 1.81 (d, *J* = 6.8 Hz, 3H). ¹³C NMR (100 MHz, CDCl₃): δ 153.9, 149.2, 140.3, 135.6, 135.2, 127.9, 122.5, 121.2, 120.1, 119.7, 115.7, 115.4, 110.1, 77.3, 59.8, 56.4, 45.7, 34.0, 19.3. HRMS (ESI) (m/z): [M+H]⁺calculated for C₂₂H₂₅ClFN₅O₂, 446.1714; found 446.1735. HPLC purity: 96.00%, retention time = 10.530 min.

### Example 5 WWQ-189 (prepared with (S)-1-(2,6-dichloro-3-fluorophenyl)ethanol as raw material)

### (R)-5-[1-(2,6-dichloro-3-fluorophenyl)ethoxy]-[3,3'-dipyridine]-6-amine

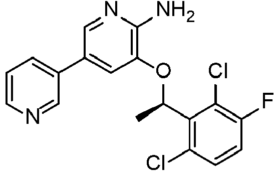

83 mg of brown solids were obtained with a yield of 55.6%, melting point: 158.8-159.8 °C. ¹H NMR (400 MHz, CDCl₃): δ 8.55 (s, 1H), 8.55 (d, *J=* 4.0 Hz, 1H), 7.88 (s, 1H), 7.69-7.66 (m, 1H), 7.34-7.30 (m, 2H), 7.07 (t, *J=* 8.0 Hz, 1H), 6.98 (d, *J* = 2.0 Hz, 1H), 6.13 (q, *J* = 6.8 Hz, 1H), 5.05 (s, 2H), 1.88 (d, *J=* 6.8 Hz, 3H). ¹³C NMR (100 MHz, CDCl₃): δ 150.1, 148.1, 147.3, 140.0, 136.6, 136.2, 134.4, 133.8, 133.3, 130.0, 129.0, 123.7, 123.6, 122.2, 122.0, 117.0, 116.8, 115.7, 72.8, 18.9. HRMS (ESI) (m/z): [M+H]⁺ calculated for C₁₈H₁₄Cl₂FN₃O, 378.0532; found 378.0570. HPLC purity: 95.50%, retention time = 10.510 min.

### Example 6 WWQ-191 (prepared with (S)-1-(2,6-dichloro-3-fluorophenyl)ethanol as raw material)

### (R)-5-[1-(2,6-dichloro-3-fluorophenyl)ethoxy]-6'-piperazin-[3,3'-dipyridine]-6-amine

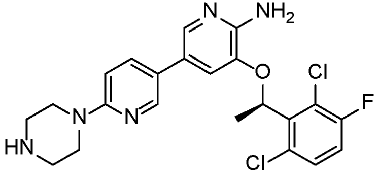

78 mg of light brown solids were obtained with a yield of 46.0%, melting point: 197.8-198.5 °C. ¹H NMR (400 MHz, CDCl₃): δ 8.23 (s, 1H), 7.78 (s, 1H), 7.50 (d, *J=* 8.4 Hz, 1H), 7.32-7.28 (m, 2H), 7.05 (t, *J*= 8.4 Hz, 1H), 6.92 (s, 1H), 6.67 (d, *J* = 8.4 Hz, 1H), 6.11 (q, *J*= 6.4 Hz, 1H), 4.84 (s, 2H), 3.56 (s, 4H), 3.03 (s, 4H), 1.86 (d, *J=* 6.4 Hz, 3H). ¹³C NMR (100 MHz, CDCl₃): δ 158.6, 149.3, 145.3, 139.9, 137.0, 136.4, 135.3, 129.0, 124.6, 123.7, 122.1, 121.9, 116.8, 116.6, 115.2, 107.0, 72.4, 46.3, 45.8, 18.9.HRMS (ESI) (m/z): [M+H]⁺ calculated for C₂₂H₂₂Cl₂FN₅O, 462.1219; found 462.1265. HPLC purity: 97.82%, retention time = 9.935 min.

### Example 7 WWQ-197

### 4-{3-[1-cyclohexaneethoxy]-2-aminopyridine }pyrazol-1-piperidine

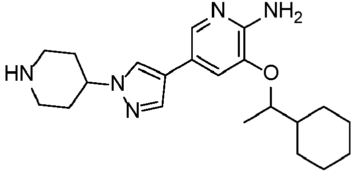

78 mg of yellow solids were obtained with a yield of 52.3%, melting point: 192.2-193.4 °C. ¹H NMR (400 MHz, CDCl₃): δ 7.77 (d, *J* = 2.0 Hz, 1H), 7.66 (s, 1H), 7.59 (s, 1H), 6.96 (d, *J* = 1.6 Hz, 1H), 4.64 (s, 2H), 4.27-4.17 (m, 2H), 3.26 (d, *J* = 12.8 Hz, 2H), 2.78 (td, *J*₁ = 2.4 Hz, *J*₂ = 12.8 Hz, 2H), 2.19 (dd, *J*₁ = 2.4 Hz, *J*₂ = 12.4 Hz, 2H), 1.81-1.62 (m, 4H), 1.28 (d, *J* = 6.8 Hz, 3H), 1.24-1.05 (m, 4H). ¹³C NMR (100 MHz, CDCl₃):8 149.6, 140.9, 135.9, 135.2, 122.8, 120.2, 119.5, 115.6, 78.5, 59.8, 45.7, 43.2, 34.0, 28.9, 28.5, 26.5, 26.1, 16.5. HRMS (ESI) (m/z): [M+H]⁺calculated for C₂₁H₃₁N₅O, 370.2562; found 370.2601. HPLC purity: 95.58%, retention time = 10.841 min.

### Example 8 Preparation of WWQ-1093-13

### 4-{3-[1-(2,6-dichloro-3-fluorophenyl)propoxy]-2-aminopyridine}pyrazol-1-piperidine

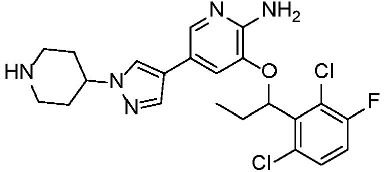

110 mg of light yellow solids were obtained with a yield of 60.2%, melting point: 196.6-197.1 °C. ¹H NMR (400 MHz, CDCl₃): δ 7.78 (s, 1H), 7.56 (s, 1H), 7.49 (s, 1H), 7.34 (dd, *J*₁ = 2.4 Hz, *J*₂ = 6.0 Hz, 1H), 7.25 (dd, *J*₁ = 2.8 Hz, *J*₂ = 5.6 Hz, 1H), 6.79 (s, 1H), 5.41 (t, *J=* 6.4 Hz, 1H), 4.74 (s, 2H), 4.26-4.20 (m, 1H), 3.30 (d, *J* = 12.8 Hz, 2H), 2.80 (t, *J* = 12.0 Hz, 2H), 2.44 (s, 1H), 2.23 (d, *J* = 10.8 Hz, 2H), 2.09-1.92 (m, 4H), 1.05 (t, *J=* 7.2 Hz, 3H). ¹³C NMR (100 MHz, CDCl₃): δ 148.8, 139.9, 136.3, 130.2, 129.9, 125.5, 122.8, 119.7, 119.5, 115.6, 74.4, 59.5, 45.4, 33.5, 29.9, 9.8.HRMS (ESI) (m/z): [M+H]⁺ calculated for C₂₂H₂₄Cl₂FN₅O, 464.1375; found 464.1418. HPLC purity: 96.85%, retention time = 11.222 min.

### Example 9 Preparation of WWQ-1093-23

### (R)-5-[1-(2,6-dichloro-3-fluorophenyl)ethoxy]-5'-piperazine-[3,3'-dipyridine]-6-amine

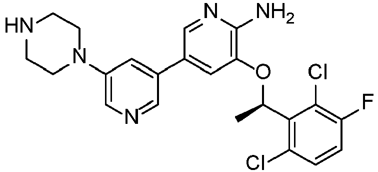

36 mg of white solids were obtained with a yield of 49.2%, melting point: 189.7-190.6 °C. ¹H NMR (400 MHz, CDCl₃): δ 8.22 (d, *J* = 2.4 Hz, 1H), 8.13 (d, *J* = 2.0 Hz, 1H), 7.86 (d, *J* = 1.6 Hz, 1H), 7.30 (dd, *J*₁ = 4.8 Hz, *J*₂ = 8.8 Hz, 1H), 7.09-7.04 (m, 2H), 6.93 (d, *J =* 2.4 Hz, 1H), 6.10 (q, *J* = 6.4Hz, 1H), 4.96 (s, 2H), 3.25-3.22 (m, 4H), 3.11-3.08 (m, 4H), 2.30 (s, 1H), 1.87 (d, *J*= 6.8 Hz, 3H). ¹³C NMR (100 MHz, CDCl₃): δ 149.1, 142.2, 140.6, 135.9, 135.6, 128.9, 128.0, 125.4, 122.8, 120.1, 119.3, 116.7, 77.2, 59.2, 45.2, 33.2, 24.3. HRMS (ESI) (m/z): [M+H]⁺ calculated for C₂₂H₂₂Cl₂FN₅O, 462.1219; found 462.1268. HPLC purity: 91.95%, retention time = 9.311 min.

### Example 10 Preparation of WWQ-1093-25

### 4-[3-(1-phenylpropoxy)-2-aminopyridine]pyrazole-1-piperidine

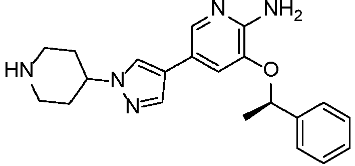

56 mg of light yellow solids were obtained with a yield of 53.4%, melting point: 194.2-195.0 °C. ¹H NMR (400 MHz, CDCl₃): δ 7.74 (d, *J* = 1.6 Hz, 1H), 7.48 (s, 1H), 7.44 (s, 1H), 7.37-7.34 (m, 4H), 7.31-7.29(m, 1H), 6.83 (d, *J=* 1.6 Hz, 1H), 5.34 (q, *J=* 6.4 Hz, 1H), 4.74 (s, 2H), 4.25-4.18 (m, 1H), 3.28 (d, *J* = 12.0 Hz, 2H), 2.80 (t, *J* = 11.6 Hz, 2H), 2.62 (s, 1H), 2.16 (d, *J* = 11.2 Hz, 2H), 1.94 (q, *J* = 3.2Hz, 2H), 1.69 (d, *J* = 2.4 Hz, 3H). ¹³C NMR (100 MHz, CDCl₃): δ 149.1, 142.2, 140.6, 135.9, 135.6, 128.9, 128.0, 122.8, 120.1, 119.3, 116.7, 77.2, 59.2, 45.2, 33.2, 24.3. HRMS (ESI) (m/z): [M+H]⁺ calculated for C₂₁H₂₅N₅O, 364.2093; found 364.2133. HPLC purity: 95.93%, retention time = 9.597 min.

### Example 11 Preparation of WWQ-1093-27

### 4-{3-[1-(2,6-dichloro-3-fluorophenyl)ethoxy]-2-aminopyridine}pyrazole-1-morpholine

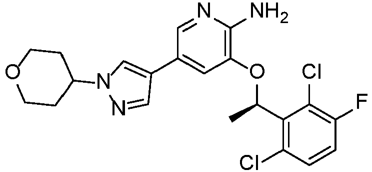

37 mg of light brown solids were obtained with a yield of 53.0%, melting point: 188.1-190.4°C. ¹H NMR (400 MHz, CDCl₃): δ 7.74 (d, *J* = 2.0 Hz, 1H), 7.58 (s, 1H), 7.51 (s, 1H), 7.31 (dd, *J* = 4.8 Hz, 1H), 7.06(t, *J* = 8 Hz, 1H), 6.89 (d, *J* = 1.6 Hz, 1H), 6.08 (q, *J* = 6.8 Hz, 1H), 4.98 (s, 2H), 4.38-4.31 (m, 1H), 4.12 (d, *J* = 11.6 Hz, 2H), 3.56 (td, *J*₁ = 3.2 Hz, *J*₂ = 11.6 Hz, 2H), 2.12-2.08 (m, 4H), 1.87 (d, *J=* 6.8 Hz, 3H). ¹³C NMR (100 MHz, CDCl₃): δ 140.2, 136.6, 136.5, 135.8, 132.5, 122.7, 119.4, 119.1, 117.1, 116.8, 115.6, 77.2, 66.8, 58.3, 33.4, 29.7, 24.9, 18.9. HRMS (ESI) (m/z): [M+H]⁺ calculated for C₂₁H₂₁Cl₂FN₄O₂, 451.1059; found 451.1100. HPLC purity: 99.22%, retention time = 12.897 min.

### Example 12 Preparation of WWQ-1093-65

### 4-{3-[1-(2,6-dichloro-3-fluorophenyl)ethoxy]-2-aminopyridine}pyrazole-1-piperidine

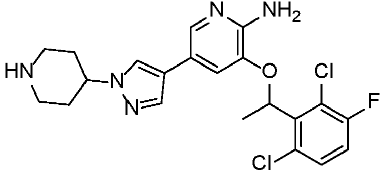

41 mg of white solids were obtained with a yield of 50.3%, melting point: 189.6-190.9°C. ¹H NMR (400 MHz, CDCl₃): δ 7.94 (s, 1H), 7.78 (s, 1H), 7.58 (s, 2H), 7.45 (s, 1H), 6.91(s, 1H), 6.09 (d, *J* = 2.4 Hz, 1H), 5.77 (s, 1H), 5.70 (s, 2H), 4.39 (s, 1H), 2.95 (t, *J* = 3.6 Hz, 2H), 2.11-2.04 (m, 4H), 1.80 (d, *J =* 6.4 Hz, 3H), 0.84 (t, *J* = 2.4Hz, 2H). ¹³C NMR (100 MHz, CDCl₃): δ 148.9, 139.8, 136.9, 135.6, 135.5, 129.0, 128.9, 122.5, 119.9, 119.3, 116.8, 116.6, 114.9, 72.4, 59.8, 45.7, 33.9, 18.9. HRMS (ESI) (m/z): [M+H]⁺ calculated for C₂₁H₂₂Cl₂FN₅O, 450.1219; found 450.1260. HPLC purity: 97.77%, retention time = 10.460 min.

### Example 13 Preparation of compound WWQ-1093-71

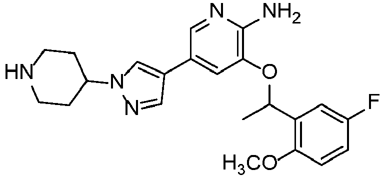

27 mg of yellow solids were obtained with a yield of 35.1%, melting point: 191.6-192.3°C. ¹H NMR (400 MHz, DMSO-*d*₆): δ 7.98 (s, 1H), 7.76 (d, *J=* 2.0 Hz, 1H), 7.67 (s, 1H), 7.32 (dd, *J*₁ = 2.8, *J* ₂= 9.6 Hz, 1H), 7.08-7.04 (m, 3H), 6.01 (s, 2H), 5.82 (q, *J* = 6.0 Hz, 1H), 4.49-4.44 (m, 1H), 3.89 (s, 3H), 3.78-3.37 (m, 2H), 3.06 (td, *J*₁ = 4.8 Hz, *J* ₂= 7.6 Hz, 2H), 2.20-2.14 (m, 4H), 1.56 (d, *J=* 6.4 Hz, 3H), 1.23 (s, 1H). ¹³C NMR (100 MHz, DMSO-*d*₆): δ 157.8, 155.5, 152.1, 149.6, 139.4, 135.3, 131.9, 124.1, 119.2, 117.2, 115.4, 114.9, 114.6, 112.9, 112.6, 112.4, 68.8, 56.3, 55.1, 28.5, 21.9.

HRMS (ESI) (m/z): [M+H]⁺calculated for C₂₂H₂₆FN₅O₂, 412.2104; found 412.2148. HPLC purity: 97.44%, retention time = 10.154 min.

### Example 14 Preparation of WWQ-1093-73

### 4-{3-[1-(2,6-dichloro-3-fluorophenyl)ethylamino]-2-aminopyridine}pyrazole-1-piperidine

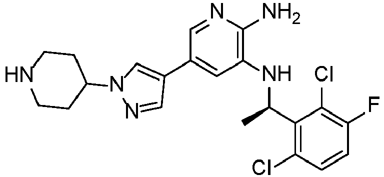

38 mg of light brown solids were obtained with a yield of 40.2%, melting point: 191.5-191.7°C. ¹H NMR (400 MHz, CDCl₃): δ 7.78 (s, 1H), 7.48 (s, 1H), 7.44 (s, 1H), 7.36 (t, *J=* 8.8 Hz, 1H), 6.29 (d, *J* = 1.6Hz, 1H), 5.77 (s, 2H), 5.65 (d, *J* = 7.6 Hz, 1H), 5.28-5.23 (m, 1H), 4.36-4.29 (m, 1H), 3.24 (d, *J* = 12.8 Hz, 2H), 2.89 (t, *J* = 10.8 Hz, 2H), 2.11-1.94 (m, 4H), 1.68 (d, *J* = 7.2 Hz, 3H). ¹³C NMR (100 MHz, CDCl₃): δ 146.9, 139.4, 134.6, 131.0, 128.3, 123.1, 120.0, 117.3, 110.8, 58.4, 43.0, 30.3, 18.4. HRMS (ESI) (m/z): [M+H]⁺ calculated for C₂₁H₂₃Cl₂FN₆, 449.1379; found 449.1424. HPLC purity: 97.37%, retention time = 10.300 min.

### Example 15 Preparation of WWQ-1093-75

### 3-(1-(2-chloro-5-fluorophenyl)ethoxy)-2-amino-5-phenylpyridine

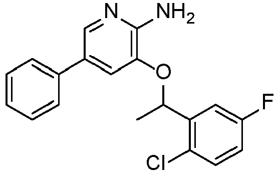

67 mg of yellow solids were obtained with a yield of 51.7%, melting point: 199.0-199.8°C. ¹H NMR (400 MHz, CDCl₃): δ 7.89 (s, 1H), 7.39-7.34 (m, 5H), 7.28 (d, *J=* 6.8 Hz,1H), 7.17 (dd, *J*₁ *=* 2.4 Hz, *J*₂ = 8.8 Hz, 1H), 6.96-6.91 (m, 1H), 6.88 (s, 1H), 5.73 (q, *J* = 6.0 Hz, 1H), 4.91 (s, 2H), 1.68 (d, *J* = 6.4 Hz, 3H). ¹³C NMR (100 MHz, CDCl₃): δ 163.3, 160.8, 149.3, 141.7, 140.0, 138.2, 137.0, 131.1, 129.0, 127.4, 127.0, 126.2, 116.8, 116.5, 116.3, 113.7, 113.5, 72.7, 22.5. HRMS (ESI) (m/z): [M+H]⁺ calculated for C₁₉H₁₆ClFN₂O, 343.0969; found 343.1011. HPLC purity: 96.54%, retention time = 14.294 min.

### Example 16 Preparation of WWQ-1093-81

### 3-(1-(2-chloro-5-fluorophenyl)ethoxy)-2-amino-5-pyrazolepyridine

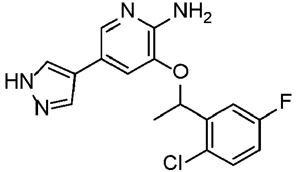

67 mg of light yellow solids were obtained with a yield of 58.8%, melting point: 183.8-184.6°C. ¹H NMR (400 MHz, CDCl₃): δ 7.80 (s, 1H), 7.66 (s, 2H), 7.37 (d, *J* = 4.8 Hz, 1H), 7.15 (dd, *J*₁ = 2.8 Hz, *J*₂ = 8.8 Hz, 1H), 6.99-6.92 (m, 1H), 6.77 (s, 1H), 5.71 (q, *J=* 6.0 Hz, 1H), 5.06 (s, 2H), 1.68 (d, *J= 6.4* Hz, 3H). ¹³C NMR (100 MHz, CDCl₃): δ 163.3, 160.9, 148.7, 141.7, 140.1, 135.5, 131.1, 131.0, 126.2, 126.1, 116.6, 116.4, 116.1, 113.8, 113.5. HRMS (ESI) (m/z): [M+H]⁺ calculated for C₁₆H₁₄ClFN₄O, 333.0874; found 333.0919. HPLC purity: 96.37%, retention time = 11.513 min.

### Example 17 Preparation of compound WWQ-1093-83

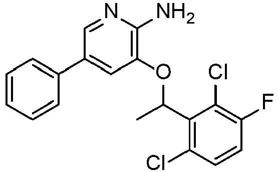

136 mg of light brown solids were obtained with a yield of 60.4%, melting point: 160.4-161.4°C. ¹H NMR (400 MHz, CDCl₃): δ 7.87 (d, *J* = 1.6 Hz, 1H), 7.21-7.36 (m, 4H), 7.32-7.27 (m, 2H), 7.06 (t, *J* = 8.4 Hz, 1H), 7.23 (d, *J =* 1.6 Hz, 1H), 6.13 (q, *J* = 6.8 Hz, 1H), 4.99 (s, 2H), 1.86 (d, *J =* 6.8 Hz, 3H). ¹³C NMR (100 MHz, CDCl₃): δ 149.6, 139.8, 138.2, 136.9, 136.6, 130.0, 129.0, 128.9, 127.2, 126.9, 126.1, 122.2, 122.0, 116.8, 116.6, 116.1, 72.6, 18.9. HRMS (ESI) (m/z): [M+H]⁺calculated for C₁₉H₁₅Cl₂FNO, 377.0579; found 377.0623. HPLC purity: 99.02%, retention time = 14.543 min.

### Example 18 Preparation of compound WWQ-1093-85

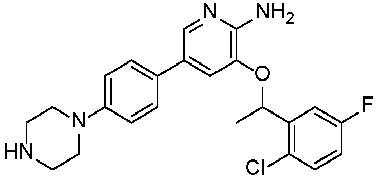

45 mg of light brown solids were obtained with a yield of 50.2%, melting point: 191.1-193.7°C. ¹H NMR (400 MHz, CDCl₃): δ 7.84 (s, 1H), 7.36-7.33 (m, 1H), 7.27 (s, 1H), 7.25 (s, 1H), 7.16 (dd, *J*₁ = 2.0, *J* ₂= 8.8 Hz, 1H), 6.92 (d, *J* = 8.0 Hz, 3H), 6.84 (s, 1H), 5.71 (q, *J* = 6.4 Hz, 1H), 4.79 (s, 2H), 3.17 (d, *J* = 4.8 Hz, 4H), 3.06 (d, *J* = 4.8 Hz, 4H), 2.29 (s, 1H), 1.67 (d, *J* = 6.4 Hz, 3H). ¹³C NMR (100 MHz, CDCl₃): δ 163.3, 160.8, 150.5, 148.8, 141.8, 139.9, 136.7, 131.0, 130.9, 129.8, 127.3, 126.9, 126.2, 116.5, 116.2, 113.7, 113.5, 72.6, 49.8, 45.7, 22.5. HRMS (ESI) (m/z): [M+H]⁺ calculated for C₂₃H₂₄ClFN₄O, 427.1656; found 427.1700. HPLC purity: 96.10%, retention time = 10.662 min.

### Example 19 Preparation of compound WWQ-1093-87

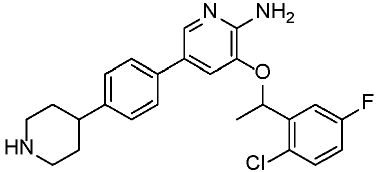

43 mg of light brown solids were obtained with a yield of 54.1%, melting point: 189.8-191.0°C. ¹H NMR (400 MHz, CDCl₃): δ 7.88 (s, 1H), 7.36 (dd, *J*₁ = 4.4 Hz, *J*₂ = 8.8 Hz, 1H), 7.31-7.22 (m, 4H),7.16 (d, *J* = 6.8 Hz, 1H), 6.94 (t, *J=* 7.6 Hz, 1H), 6.88 (s, 1H), 5.72 (q, *J* = 6.4 Hz, 1H), 4.87 (s, 2H), 3.29 (d, *J* = 12.0 Hz, 2H), 3.06 (s, 1H), 2.81 (t, *J* = 8.0 Hz, 2H), 2.66 (t, *J* = 11.6 Hz, 1H), 1.90-1.75 (m, 4H), 1.67 (d, *J*= 6.4 Hz, 3H). ¹³C NMR (100 MHz, CDCl₃): δ 163.2, 160.8, 149.3, 144.4, 141.8, 140.0, 137.2, 136.4, 131.0, 127.3, 126.6, 116.6, 116.5, 116.3, 113.7, 113.4, 72.6, 46.3, 41.9, 33.0, 29.7, 22.5. HRMS (ESI) (m/z): [M+H]⁺calculated for C₂₄H₂₅ClFN₃O, 426.1704; found 426.1746. HPLC purity: 97.45%, retention time = 10.951 min.

### Example 20 Preparation of compound WWQ-1093-89

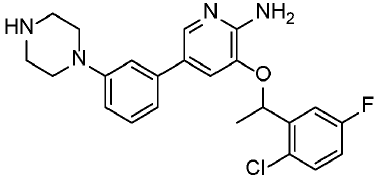

45 mg of light yellow solids were obtained with a yield of 55.1%, melting point: 190.8-191.6°C. ¹H NMR (400 MHz, CDCl₃): δ 7.86 (s, 1H), 7.36 (dd, *J*₁ = 5.2 Hz, *J*₂ = 8.8 Hz, 1H), 7.29-7.24 (m, 1H), 7.16 (dd, *J*₁ = 2.4 Hz, *J*₂ = 8.8 Hz, 1H), 6.96-6.93 (m, 5H), 5.71 (q, *J=* 6.4 Hz, 1H), 5.62 (s, 1H), 4.90 (s, 2H), 3.38 (d, *J=* 4.4 Hz, 4H), 3.28 (d, *J=* 4.0 Hz, 4H), 1.67 (d, *J=* 6.0 Hz, 3H). ¹³C NMR (100 MHz, CDCl₃): δ 163.3, 160.8, 151.0, 149.4, 141.9, 139.8, 139.4, 137.2, 131.0, 129.8, 127.3, 126.2, 118.9, 116.8, 115.4, 114.8, 113.8, 72.6, 48.1, 44.3, 29.7, 22.6. HRMS (ESI) (m/z): [M+H]⁺ calculated for C₂₃H₂₄ClFN₄O, 427.1656; found 427.1700. HPLC purity: 97.55%, retention time = 10.854 min.

### Example 21 Preparation of compound WWQ-1093-91

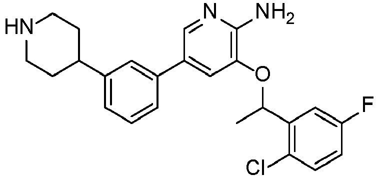

43 mg of light yellow solids were obtained with a yield of 50.3%, melting point: 192.1-193.4°C. ¹H NMR (400 MHz, CDCl₃): δ 7.87 (s, 1H), 7.38 (dd, *J*₁ = 5.2 Hz, *J*₂ = 8.8 Hz, 1H), 7.32 (t, *J=* 8.4 Hz, 1H), 7.22-7.15 (m, 4H), 7.30 (td, *J*₁ = 2.8 Hz, *J*₂ = 8.4 Hz, 1H), 6.86 (s, 1H), 5.74 (q, *J* = 6.4Hz, 1H), 4.90 (s, 2H), 3.57 (d, *J* = 8.4 Hz, 2H), 3.00 (t, *J* = 12.4 Hz, 2H), 2.76 (t, *J* = 11.6 Hz, 2H), 2.14-2.01 (m, 4H), 1.87 (d, *J*= 6.4 Hz, 3H). ¹³C NMR (100 MHz, CDCl₃): δ 163.3, 160.8, 149.4, 144.4, 141.8, 139.8, 138.8, 137.3, 131.1, 129.4, 127.1, 126.2, 125.1, 116.7, 116.4, 113.8, 113.5, 72.6, 44.7, 40.9, 30.4, 22.6. HRMS (ESI) (m/z): [M+H]⁺ calculated for C₂₄H₂₅ClFN₃O, 426.1704; found 426.1746. HPLC purity: 95.06%, retention time = 11.077 min.

MXY series of compounds were synthesized according to the following synthetic route:

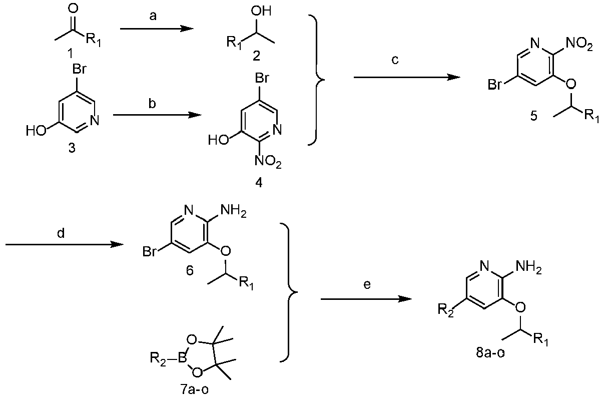

Compound 193 is taken as an example:

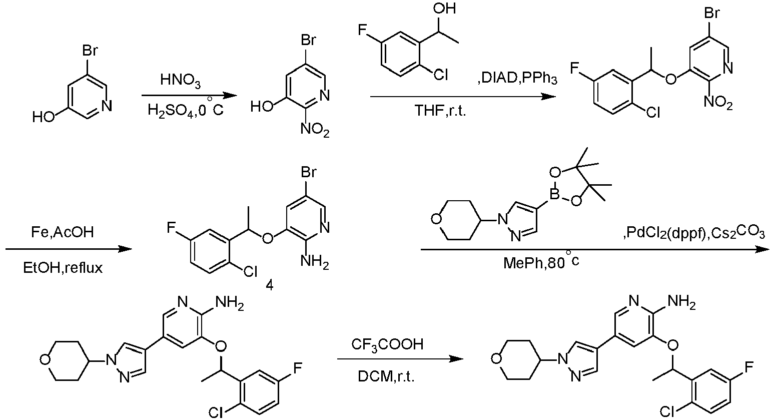

Reagents and conditions: (a) NaBH₄, MeOH, 0°C; (b) H₂SO₄, HNO₃, 0°C; (c) PPh₃, DIAD, THF, 0°C; (d) 1:1 CH₃COOH: CH₃CH₂OH, Fe, 78°C; (e) Cs₂CO3, Pd(dppf)Cl₂, toluene, H₂O, 80°C.

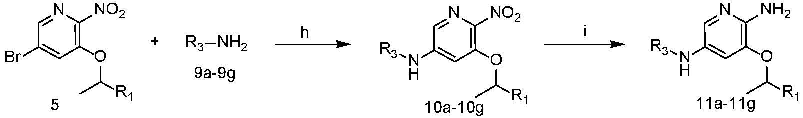

Reagents and conditions: (h) Pd(OAc)2, 4,5-bis(diphenylphosphine)-9,9-dimethylxanthene (Xantphos), dioxane, 100°C, 12 h; (i) 1:1 CH₃COOH: CH₃CH₂OH, Fe, 78°C.

### Example 22 Preparation of compound MXY-89

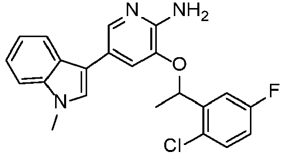

68 mg of white solids were obtained with a yield of 60.8%. ¹H NMR (400 MHz, CDCl₃) δ ppm 7.89 (d, *J* = 1.56 Hz, 1H), 7.36 (dd, *J* = 8.67, 4.66 Hz, 2H), 7.30 (d, *J* = 8.24 Hz, 1H), 7.24-7.18 (m, 2H), 7.09-7.03 (m, 2H), 6.98-6.93 (m, 2H), 5.70 (q, *J* = 6.28 Hz, 1H), 4.82 (s, 2H), 3.77 (s, 3H), 1.69 (d, *J* = 6.35 Hz, 3H). ¹³C NMR (100 MHz, DMSO-*d6*) δ ppm 162.0, 149.8, 142.8, 139.2, 137.4, 137.4, 137.0, 131.8, 126.7, 126.5, 125.8, 121.9, 120,4, 119.7, 119.1, 117.09, 114.3, 112.7, 110.5, 72.0, 32.9, 22.5.HRMS (EI) m/z: calculated for C₂₂H₁₉ClFN₃O 395.1201; found 395.1198.

### Example 23 Preparation of compound MXY-97

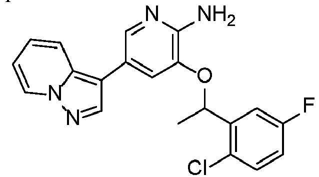

67 mg of light brown solids were obtained with a yield of 60.8%. ¹H NMR (400 MHz, CDCl₃□□δ ppm 8.43 (d, *J* = 7.03 Hz, 1H), 7.92 (s, 1H), 7.87 (d, *J* = 1.57 Hz, 1H), 7.39-7.33 (m, 2H), 7.18 (dd, *J* = 9.05, 3.02 Hz, 1H), 7.09-7.03 (m, 1H), 6.99-6.93 (m, 1H), 6.81 (d, *J* = 1.54 Hz, 1H), 6.74 (t, *J* = 6.50 Hz, 1H), 5.71 (q, *J* = 6.29 Hz, 1H), 4.91 (s, 2H), 1.69 (d, *J* = 6.35 Hz, 3H). ¹³C NMR (100 MHz, DMSO-*d6*) δ ppm 161.9, 150.3, 142.7, 139.8, 139.2, 137.2, 136.1, 131.7, 129.7, 126.7, 124.4, 117.5, 117.2, 177.1, 116.7, 114.5, 112.7, 109.8, 71.9, 22,5. HRMS (EI) m/z: calculated for C₂₀H₁₆ClFN₄O 382.0097; found 382.0998.

### Example 24 Preparation of compound MXY-111

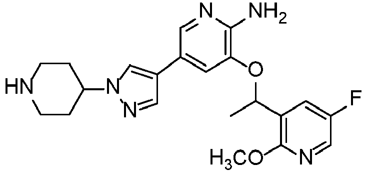

White solids were obtained with a yield of 76.2%. ¹H NMR (400 MHz, DMSO-*d6*□□□ ppm 8.09 (d, *J* = 2.99 Hz, 1H), 8.03 (s, 1H), 7.95 (dd, *J* = 8.79, 2.94 Hz, 1H), 7.79 (d, *J* = 1.72 Hz, 1H), 7.66 (s, 1H), 7.17 (d, *J* = 1.54 Hz, 1H), 5.84 (s, 2H), 5.72 (q, *J* = 6.06 Hz, 1H), 4.20-4.10 (m, 1H), 3.96 (s, 3H), 3.03 (d, *J* = 12.37 Hz, 2H), 2.58 (dd, *J* = 11.97, 10.75 Hz, 2H), 1.95 (dd, *J* = 11.60, 1.88 Hz, 2H), 1.77 (qd, *J* = 12.04, 3.95 Hz, 2H), 1.56 (d, *J* = 6.26 Hz, 3H). ¹³C NMR (100 MHz, DMSO-*d6*) δ ppm 156.7, 155.9, 154.7, 150.4, 139.3, 136.0, 135.1, 132.5, 126.6, 124.1, 119.6, 118.1, 116.0, 69.4, 59.7, 54.4, 45.6, 34.1, 21.6. HRMS (EI) m/z: calculated for C₂₁H₂₅FN₆O₂ 412.2023; found 412.2021.

### Example 25 Preparation of compound MXY-137

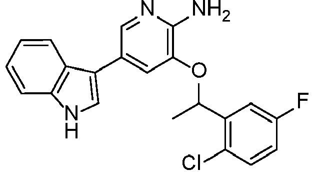

Light brown solids were obtained with a yield of 48.3%. ¹H NMR (400 MHz, DMSO-*d6*□□□ ppm 11.18 (s, 1H), 7.78 (d, *J* = 1.79 Hz, 1H), 7.58 (dd, *J* = 8.86, 5.08 Hz, 1H), 7.47 (dd, *J* = 9.56, 3.12 Hz, 1H), 7.42 (d, *J* = 2.49 Hz, 1H), 7.39 (d, *J* = 8.12 Hz, 1H), 7.28 (d, *J* = 7.99 Hz, 1H), 7.26-7.20 (m, 1H), 7.13-7.08 (m, 1H), 7.00-6.97 (m, 1H), 6.97-6.95 (m, 1H), 5.84 (s, 2H), 5.81-5.76 (m, 1H), 1.64 (d, *J* = 6.32 Hz, 3H). ¹³C NMR (100 MHz, DMSO-*d6*) δ ppm 162.0, 149.7, 142.8, 139.1, 137.1, 137.0, 131.8, 126.7, 125.4, 122.2, 121.8, 120.8, 119.6, 118.9, 117.2, 117.0, 114.3, 113.5, 112.3, 72.0, 22.5. HRMS (EI) m/z: calculated for C₂1H₁₇ClFN₃O 381.1044; found 381.1045.

### Example 26 Preparation of compound MXY-141

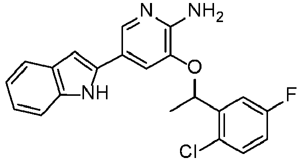

Light brown solids were obtained with a yield of 51.2%. ¹H NMR (400 MHz, DMSO-*d6*□□□ ppm 11.31 (s, 1H), 8.05 (d, *J* = 1.73 Hz, 1H), 7.62 (dd, *J* = 9.69, 3.10 Hz, 1H), 7.53 (dd, *J* = 8.85, 5.11 Hz, 1H), 7.46 (d, *J* = 7.74 Hz, 1H), 7.33 (d, *J* = 7.99 Hz, 1H), 7.30 (d, *J* = 1.63 Hz, 1H), 7.19 (td, *J* = 8.52, 3.12 Hz, 1H), 7.06-7.01 (m, 1H), 6.98-6.93 (m, 1H), 6.53 (d, *J* = 1.51 Hz, 1H), 6.19 (s, 2H), 5.89 (q, *J* = 6.11 Hz, 1H), 5.76 (s, 1H), 1.63 (d, *J* = 6.26 Hz, 3H). ¹³C NMR (100 MHz, DMSO-*d*₆): δ 161.9, 151.5, 142.6, 139.2, 137.2, 136.8. 136.6, 131.6, 129.1, 126.7, 121.3, 119.8, 119.7, 117.5, 117.1, 115.3, 114.7, 111.3, 96.9, 72.0, 22.2. HRMS (EI) m/z: calculated for C₂1H₁₇ClFN₃O 381.1044; found 381.1042.

### Example 27 Preparation of compound MXY-171

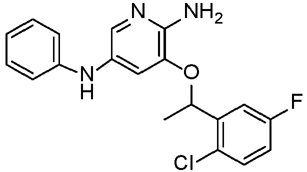

White solids were obtained with a yield of 37.2%. ¹H NMR (400 MHz, DMSO-*d6*□□□□ppm 8.56 (s, 1H), 8.04-7.90 (m, 3H), 7.59 (d, *J* = 2.09 Hz, 1H), 7.55 (dd, *J* = 8.85, 5.08 Hz, 1H), 7.45-7.37 (m, 3H), 7.19 (td, *J* = 8.52, 3.14 Hz, 1H), 6.67-6.56 (m, 1H), 6.52 (d, *J* = 8.42 Hz, 1H), 5.64 (q, *J* = 6.20 Hz, 1H), 5.51 (s, 2H), 1.60 (d, *J* = 6.31 Hz, 3H). ¹³C NMR (100 MHz, DMSO-*d6*) δ ppm 161.9, 147.4, 146.3, 142.5, 139.0, 132.1, 131.8, 129.4, 129.3, 126.7, 118.1, 117.0, 114.2, 113.9, 113.8, 72.0, 66.8, 22.4. HRMS (EI) m/z: calculated for C₁₉H₁₇ClFN₃O 357.1044; found 357.1049.

### Example 28 Preparation of compound MXY-175

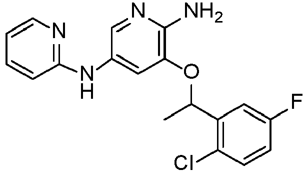

White solids were obtained with a yield of 41.7%. ¹H NMR (400 MHz, DMSO-*d6□*□□ ppm 8.57 (s, 1H), 7.97 (dd, *J* = 5.00, 1.29 Hz, 1H), 7.59 (d, *J* = 2.07 Hz, 1H), 7.55 (dd, *J* = 8.85, 5.08 Hz, 1H), 7.44 (d, *J* = 1.97 Hz, 1H), 7.43-7.37 (m, 2H), 7.19 (td, *J* = 8.53, 3.14 Hz, 1H), 6.72-6.55 (m, 1H), 6.53 (d, *J* = 8.42 Hz, 1H), 5.64 (q, *J =* 6.19 Hz, 1H), 5.50 (s, 2H), 1.60 (d, *J* = 6.31 Hz, 3H). ¹³C NMR (100 MHz, DMSO-*d6*) δ ppm 161.8, 156.8, 147.6, 146.5, 142.7, 138.7, 137.4, 131.7, 130.1, 129.2, 126.7, 117.0, 116.8, 114.1, 113.7, 112.6, 109.7, 72.0, 22.5. HRMS (EI) m/z: calculated for C₁₈H₁₆C1FN₄O 358.0977; found 358.1000.

### Example 29 Preparation of compound MXY-181

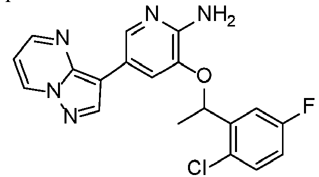

Yellow solids were obtained with a yield of 10.2%. ¹H NMR (400 MHz, DMSO-*d6□*□□ ppm 9.06 (d, *J* = *6.86* Hz, 1H), 8.53 (s, 2H), 8.19 (s, 1H), 7.70 (s, 1H), 7.53 (dd, *J* = 8.70, 5.06 Hz, 1H), 7.46 (dd, *J* = 9.50, 2.73 Hz, 1H), 7.15 (dt, *J* = 8.48, 2.72 Hz, 1H), 7.02 (dd, *J* = 6.83, 3.98 Hz, 1H), 5.95 (s, 2H), 5.80 (q, *J* = 5.67, 5.42 Hz, 1H), 1.63 (d, *J* = 6.16 Hz, 3H). ¹³C NMR (100 MHz, DMSO-*d6*) δ ppm 161.8, 150.1, 149.9, 143.9, 142.7, 142.6, 141.6, 139.1, 136.6, 131.6, 126.9, 117.1, 116.8, 116.1, 113.2, 108.9, 107.4, 72.1, 22.4. HRMS (EI) m/z: calculated for C₁₉H₁₅ClFN₅O 383.0949; found 383.0948.

### Example 30 Preparation of compound MXY-193

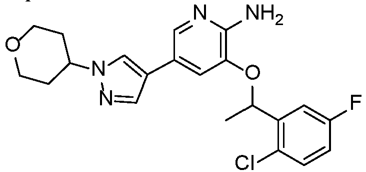

White solids were obtained with a yield of 68.9%. ¹H NMR (400 MHz, CDCl₃□□□ ppm 7.78 (d, *J* = 1.69 Hz, 1H), 7.55 (s, 1H), 7.48 (s, 1H), 7.36 (dd, *J* = 8.81, 4.96 Hz, 1H), 7.15 (dd, *J* = 9.09, 3.04 Hz, 1H), 6.94 (ddd, *J* = 8.75, 7.73, 3.05 Hz, 1H), 6.74 (d, *J* = 1.68 Hz, 1H), 5.70 (q, *J* = 6.22 Hz, 1H), 4.81 (s, 2H), 4.39-4.27 (m, 1H), 4.14-4.09 (m, 2H), 3.58-3.50 (m, 2H), 2.14-2.03 (m, 4H), 1.67 (d, *J* = 6.35 Hz, 2H). ¹³C NMR (100 MHz, CDCl₃) δ ppm 162.1, 148.7, 141.8, 139.9, 135.9, 135.8, 131.0, 126.21, 122.6, 112.0, 119.3, 116.5, 115.8, 113.7, 72.5, 66.8, 58.3, 33.3, 22.6. HRMS (EI) m/z: calculated for C₂₁H₂₂ClFN₄O₂ 416.1415; found 416.1413.

### Example 31 Preparation of compound MXY-191

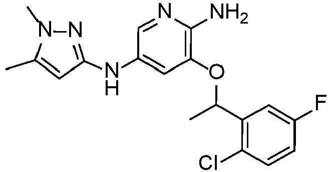

Brown solids were obtained with a yield of 43.5%. ¹H NMR (400 MHz, DMSO-*d6□*□□ ppm 7.76 (s, 1H), 7.54 (dd, *J*= 8.84, 5.08 Hz, 1H), 7.47 (d, *J*= 2.17 Hz, 1H), 7.35 (dd, *J* = 9.58, 3.12 Hz, 1H), 7.22-7.16 (m, 1H), 7.15 (d, *J*= 2.16 Hz, 1H), 5.59 (q, *J*= 6.24 Hz, 1H), 5.29 (s, 1H), 5.17 (s, 2H), 3.53 (s, 3H), 2.52-2.49 (m, 1H), 2.13 (s, 3H), 1.59 (d, *J* = 6.31 Hz, 3H). ¹³C NMR (100 MHz, DMSO-*d6*) δ ppm 161.8, 151.0, 144.2, 142.9, 142.8, 139.2, 138.8, 132.6, 131.8, 126.5, 126.4, 116.8, 114.0, 109.1, 92.7, 72.0, 35.6, 22.4, 11.1. HRMS (EI) m/z: calculated for C₁₈H₁₉ClFN₅O 375.1262; found 375.1261.

### Example 32 Preparation of compound MXY-032-45

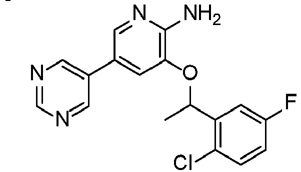

White solids were obtained with a yield of 67.4%. ¹H NMR (400 MHz, DMSO-*d6□*□□ ppm 9.06 (s, 1H), 8.95 (s, 2H), 7.98 (d, *J* = 1.78 Hz, 1H), 7.61 (m, 1H), 7.50 (dd, *J* = 8.83, 5.11 Hz, 1H), 7.30 (d, *J* = 1.57 Hz, 1H), 7.18 (dt, *J* = 8.55, 3.10 Hz, 1H), 6.35 (s, 2H), 5.94 (q, *J* = 5.96 Hz, 1H), 1.61 (d, *J* = 6.23 Hz, 3H). ¹³C NMR (100 MHz, DMSO-*d6*) δ ppm 161.8, 156.7, 153.8, 152.5, 142.5, 139.3, 138.2, 131.8, 131.6, 126.7, 117.9, 117.1, 116.1, 114.8, 72.2, 22.1. HRMS (EI) m/z: calculated for C₁₇H₁₄ClFN₄O 344.0840; found 344.0842.

### Example 33 Preparation of compound MXY-032-37

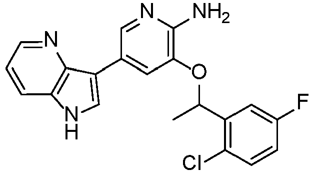

White solids were obtained with a yield of 23.5%. ¹H NMR (400 MHz, DMSO-*d6□*□□ ppm 11.41 (s, 1H), 8.35 (dd, *J*= 4.53, 1.37 Hz, 1H), 8.31 (d, *J* = 1.66 Hz, 1H), 7.90 (d, *J*= 1.52 Hz, 1H), 7.88 (s, 1H), 7.76 (dd, *J*= 8.19, 1.35 Hz, 1H), 7.55-7.45 (m, 2H), 7.18-7.09 (m, 2H), 5.85-5.77 (m, 3H), 1.64 (d, *J* = 6.30 Hz, 3H). ¹³C NMR (100 MHz, DMSO-*d6*) δ ppm 161.8, 149.5, 143.7, 142.9, 142.7, 139.0, 136.6, 131.6, 130.0, 126.9, 124.8, 120.2, 119.1, 116.9, 116.9, 116.8, 114.3, 112.5, 72.0, 22.4. HRMS (EI) m/z: calculated for C₂₀H₁₆ClFN₄O 382.0997; found 382.0995.

### Example 34 Preparation of compound MXY-032-51

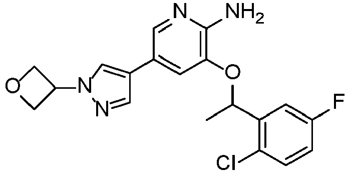

Light yellow solids were obtained with a yield of 67.8%. ¹H NMR (400 MHz, CDCl₃ □□□ ppm 7.77 (s, 1H), 7.62 (d, *J* = *3.40* Hz, 2H), 7.37 (dd, *J* = 8.70, 4.93 Hz, 1H), 7.15 (dd, *J* = 8.96, 2.68 Hz, 1H), 6.95 (dt*, J* = 8.66, 2.80 Hz, 1H), 6.75 (s, 1H), 5.71 (dd, *J* = 11.99, 5.81 Hz, 1H), 5.53-5.33 (m, 1H), 5.06 (d, *J =* 7.05 Hz, 1H), 4.93 (s, 2H), 1.68 (d, *J* = 6.27 Hz, 3H). ¹³CNMR (100 MHz, CDCl₃) δ ppm 162.1, 148.6, 141.5, 140.2, 137.2, 134.3, 131.1, 126.2, 124.1, 120.6, 118.8, 116.6, 116.1, 113.7, 77.7, 72.8, 55.7, 22.6. HRMS (EI) m/z: calculated for C₁₉H₁₈ClFN₄O₂ 388.1102; found 388.1099.

### Example 35 Preparation of compound MXY-032-53

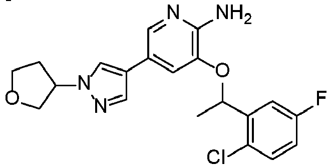

Light yellow solids were obtained with a yield of 63.9%. ¹H NMR (400 MHz, CDCl₃ □□□ ppm 7.77 (d, *J* = 1.65 Hz, 1H), 7.53 (d, *J =* 7.28 Hz, 1H), 7.36 (dd, *J* = 8.74, 4.94 Hz, 1H), 7.15 (dd, *J* = 9.07, 2.98 Hz, 1H), 6.98-6.90 (m, 1H), 6.74 (s, 1H), 5.70 (q, *J* = 6.19 Hz, 1H), 5.04-4.91 (m, 1H), 4.82 (s, 2H), 4.17-4.02 (m, 3H), 3.98-3.91 (m, 1H), 2.51-2.40 (m, 1H), 2.38-2.27 (m, 1H), 1.67 (d, *J* = 6.35 Hz, 3H). ¹³C NMR (100 MHz, CDCl₃) δ ppm 162.1, 148.6, 141.8, 140.0, 136.3, 135.6, 131.0, 126.2, 123.6, 120.5, 119.2, 116.5, 115.8, 113.7, 72.9, 72.6, 67.5, 61.9, 33.2, 22.6. HRMS (EI) m/z: calculated for C₂₀H₂₀ClFN₄O₂ 402.1259; found 402.1256.

### Example 36 Preparation of compound MXY-032-55

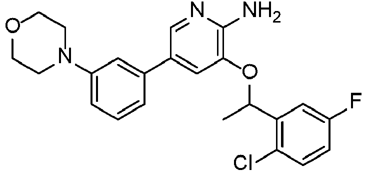

Light yellow solids were obtained with a yield of 71.8%. ¹H NMR (400 MHz, CDCl₃ □□□ ppm 7.87 (d, *J* = 1.47 Hz, 1H), 7.36-7.24 (m, 2H), 7.16 (dd, *J* = 9.04, 2.94 Hz, 1H), 6.97-6.91 (m, 1H), 6.90-6.79 (m, 4H), 5.72 (q, *J* = 6.20 Hz, 1H), 4.91 (s, 2H), 3.91-3.85 (m, 4H), 3.19-3.15 (m, 4H), 1.68 (d, *J* = 6.34 Hz, 3H). ¹³C NMR (100 MHz, CDCl₃) δ ppm 162.1, 151.7, 149.3, 141.9, 139.8, 139.2, 136.9, 130.9, 129.7, 127.7, 126.2, 118.0, 117.0, 116.4, 114.4, 113.7, 113.5, 72.7, 67.0, 49.3, 22.6. HRMS (EI) m/z: calculated for C₂₃H₂₃ClFN₃O₂ 427.1463; found 427.1466.

### Example 37 Preparation of compound MXY-02-69

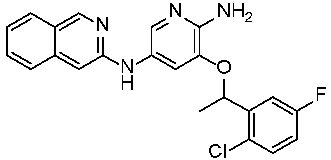

Light green solids were obtained with a yield of 34.8%. ¹H NMR (400 MHz, DMSO-*d6□*□□ ppm 8.87 (s, 1H), 8.45 (s, 1H), 7.86 (d, *J* = 8.02 Hz, 1H), 7.56 (d, *J* = 2.02 Hz, 1H), 7.54-7.49 (m, 3H), 7.46 (dd, *J* = 9.59, 3.09 Hz, 1H), 7.25-7.19 (m, 2H), 7.12 (d, *J* = 1.97 Hz, 1H), 6.66 (s, 1H), 5.68 (dd, *J* = 12.53, 6.23 Hz, 1H), 5.63 (s, 2H), 1.61 (d, *J* = 6.28 Hz, 1H). ¹³C NMR (100 MHz, DMSO-*d6*) δ ppm 161.9, 154.1, 151.7, 147.3, 142.7, 138.9, 138.6, 131.9, 131.7, 130.8, 128.6, 128.1, 126.6, 125.2, 123.7, 123.1, 117.0, 114.3, 113.7, 98.0, 72.1, 22.5. HRMS (EI) m/z: calculated for C₂₂H₁₈ClFN₄O 408.1153; found 408.1151.

### Example 38 Preparation of compound MXY-032-85

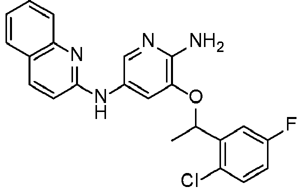

Light green solids were obtained with a yield of 41.7%. ¹H NMR (400 MHz, DMSO-*d6□*□□ ppm 9.06 (s, 1H), 7.94 (d, *J* = 8.90 Hz, 1H), 7.83 (s, 2H), 7.66 (d, *J* = 7.82 Hz, 1H), 7.56-7.46 (m, 4H), 7.26-7.18 (m, 2H), 6.87 (d, *J*= 8.90 Hz, 1H), 5.74 (dd, *J* = 11.63, 5.41 Hz, 1H), 5.60 (s, 2H), 1.64 (d, *J* = 6.22 Hz, 3H). ¹³C NMR (100 MHz, DMSO-*d6*) δ ppm 161.9, 154.8, 147.5, 146.8, 142.8, 138.6, 137.1, 131.8, 130.2, 129.7, 129.0, 127.9, 126.5, 123.8, 122.6, 116.9, 114.3, 114.0, 112.3, 72.2, 22.3. HRMS (EI) m/z: calculated for C₂₂H₁₈ClFN₄O 408.1153; found 408.1150.

### Example 39 Preparation of compound MXY-032-87

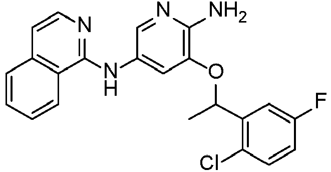

Light green solids were obtained with a yield of 43.6%. ¹H NMR (400 MHz, DMSO-*d6*□□□ ppm 8.88 (s, 1H), 8.41 (d, *J* = 8.38 Hz, 1H), 7.81-7.74 (m, 3H), 7.66 (t, *J* = 7.18 Hz, 1H), 7.60-7.52 (m, 3H), 7.47 (dd, *J* = 9.60, 3.09 Hz, 1H), 7.20 (dt, *J* = 8.52, 3.12 Hz, 1H), 7.04 (d, *J =* 5.73 Hz, 1H), 5.70-5.59 (m, 3H), 1.61 (d, *J* = 6.29 Hz, 3H). ¹³C NMR (100 MHz, DMSO-*d6*) δ ppm 161.9, 153.5, 147.3, 142.8, 141.1, 138.4, 137.3, 132.4, 131.6, 130.4, 128.4, 127.2, 126.6, 126.5, 123.5, 118.6, 116.9, 114.7, 114.3, 111.9, 72.1, 22.4. HRMS (EI) m/z: calculated for C₂₂H₁₈ClFN₄O 408.1153; found 408.1148.

### Example 40 Preparation of compound MXY-032-91

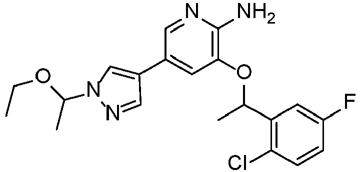

White solids were obtained with a yield of 78.6%. ¹H NMR (400 MHz, CDCl₃ □□□ ppm 7.81 (d, *J =* 1.74 Hz, 1H), 7.63 (d, *J* = 1.51 Hz, 1H), 7.55 (d, *J =* 0.67 Hz, 1H), 7.37 (dd, *J* = 8.71, 4.96 Hz, 1H), 7.19-7.14 (m, 1H), 6.98-6.91 (m, 1H), 6.76 (s, 1H), 5.71 (q, *J* = 6.28 Hz, 1H), 5.49 (q, *J* = 6.00 Hz, 1H), 4.77 (s, 2H), 3.52-3.42 (m, 1H), 3.39-3.30 (m, 1H), 1.70-1.64 (m, 6H), 1.16 (dt, *J =* 7.01, 0.76 Hz, 3H). ¹³C NMR (100 MHz, CDCl₃) δ ppm 161.9, 150.5, 142.7, 139.2, 136.3, 135.7, 131.6, 126.7, 123.8, 120.6, 117.5, 117.1, 115.9, 114.8, 86.8, 72.0, 63.5, 22.2, 21.7, 15.2. HRMS (EI) m/z: calculated for C₂₀H₂₂ClFN₄O₂ 404.1415; found 404.1413.

### Example 41 Preparation of compound MXY-032-93

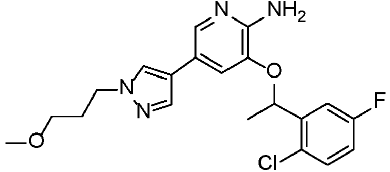

White solids were obtained with a yield of 75.9%. ¹H NMR (400 MHz, CDCl₃□□□ ppm 7.76 (d, *J =* 1.66 Hz, 1H), 7.54 (s, 1H), 7.42 (s, 1H), 7.36 (dd, *J* = 8.81, 4.96 Hz, 1H), 7.15 (dd, *J* = 9.07, 3.02 Hz, 1H), 7.04-6.87 (m, 1H), 6.74 (d, *J* = 1.68 Hz, 1H), 5.70 (q, *J* = 6.29 Hz, 1H), 4.86 (s, 2H), 4.21 (t, *J* = 6.86 Hz, 3H), 3.34-3.30 (m, 5H), 2.15-2.06 (m, 2H), 1.67 (d, *J* = 6.35 Hz, 3H). ¹³C NMR (100 MHz, CDCl₃) δ ppm 162.1, 148.6, 141.8, 140.0, 136.3, 135.7, 131.0, 126.2, 125.6, 119.8, 119.4, 116.4, 115.8, 113.6, 72.6, 69.0, 58.7, 49.0, 30.3, 22.6. HRMS (EI) m/z: calculated for C₂₀H₂₂ClFN₄O₂ 404.1415; found 404.1413.

### Example 42 Preparation of compound MXY-032-95

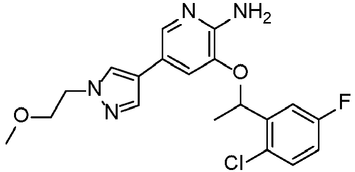

White solids were obtained with a yield of 67.9%. ¹H NMR (400 MHz, CDCl₃□□□ ppm 7.78 (d, *J =* 1.63 Hz, 1H), 7.55 (s, 1H), 7.52 (s, 1H), 7.36 (dd, *J* = 8.81, 4.96 Hz, 1H), 7.15 (dd, *J =* 9.10, 3.02 Hz, 1H), 6.97-6.88 (m, 1H), 6.74 (d, *J* = 1.65 Hz, 1H), 5.69 (q, *J* = *6.25* Hz, 1H), 4.77 (s, 2H), 4.27 (t, *J* = *5.20* Hz, 2H), 3.74 (t, *J =* 5.18 Hz, 2H), 3.34 (s, 3H), 1.67 (d, *J =* 6.35 Hz, 3H). ¹³C NMR (100 MHz, CDCl₃) δ ppm 162.1, 148.6, 141.8, 139.9, 136.3, 135.8, 131.0, 126.2, 126.1, 120.1, 119.4, 116.5, 115.8, 113.6, 72.5, 71.1, 59.0, 52.3, 22.6. HRMS (EI) m/z: calculated for C₁₉H₂₀ClFN₄O₂ 390.1259; found 390.1261.

### Example 43 Preparation of compound MXY-032-73

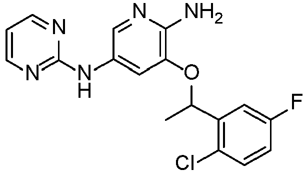

White solids were obtained with a yield of 67.9%. ¹H NMR (400 MHz, DMSO-*d6*□□□ ppm 9.20 (s, 1H), 8.31 (d, *J* = 4.41 Hz, 2H), 7.69 (s, 1H), 7.55 (dd, *J* = 8.40, 4.85 Hz, 1H), 7.49-7.37 (m, 2H), 7.22-7.15 (m, 1H), 6.71 (t, *J* = *4.29* Hz, 1H), 5.66-5.54 (m, 3H), 1.60 (d, *J* = 5.95 Hz, 3H). ¹³C NMR (100 MHz, DMSO-*d6*) δ ppm 163.1, 160.6, 158.3, 146.9, 142.7, 138.5, 131.6, 130.6, 127.9, 126.7, 116.9, 114.2, 112.7, 112.1, 72.1, 22.5. HRMS (EI) m/z: calculated for C₁₇H₁₅ClFN₅O 359.0949; found 359.0950.

### XFL series of compounds were synthesized according to the following route:

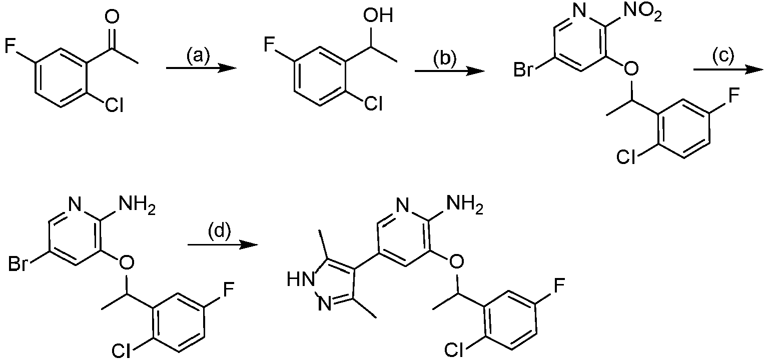

(a) NaBH₄, MeOH, 0°C- r.t. ; (b) 5-bromo-2-nitropyridin-3-ol, DIAD, PPh3, THF, 0°C - r.t. ;(c) Fe, AcOH, EtOH, reflux; (d) 3,5-dimethyl-4-(4,4,5,5-tetramethyl-1,3-dioxolane -2-y1)-1*H*-pyrazole, Cs₂CO₃, PdCl₂(dppf), dimethoxyethane, 80°C.

### Example 44 Preparation of compound XFL-107

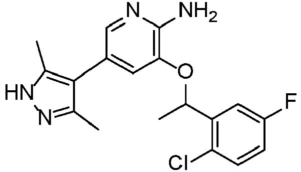

22 mg of yellow solids were obtained with a yield of 21.01%. ¹H-NMR (400 MHz, CDCl₃): *δ* 7.40 (s, 1H), 7.37 (s, 1H), 7.27 (dd, *J=* 8.8 Hz, *J=* 4.8 Hz, 1H), 7.05 (dd, *J=* 8.8 Hz, *J=* 2.8 Hz, 1H), 6.87 (td, *J*= 8.0 Hz, *J=* 3.2 Hz, 1H), 5.56 (m, 1H), 1.99 (s, 6H), 1.61 (d, *J*= 6.4 Hz, 3H). LC-MS: m/z: 361.1 (M+H)⁺.

### Example 45 Preparation of compound XFL-111

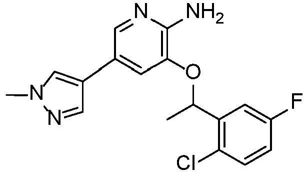

28 mg of light brown solids were obtained with a yield of 27.83%. ¹H-NMR (400 MHz, CDCl₃): *δ* 7.65 (s, 1H), 7.43 (s, 1H), 7.32 (s, 1H), 7.30 (dd, *J=* 8.8 Hz, *J=* 4.8 Hz, 1H), 7.08 (dd, *J* = 9.2 Hz, *J=* 3.2 Hz, 1H), 6.88 (td, *J=* 8.2 Hz, *J=* 3.2 Hz, 1H), 6.67 (s, 1H), 5.64 (m, 1H), 5.02 (s, 2H), 3.83 (s, 3H), 1.61 (d, *J =* 6.4 Hz, 3H). LC-MS: m/z: 347.1 (M+H)⁺.

### Example 46 Preparation of compound XFL-137

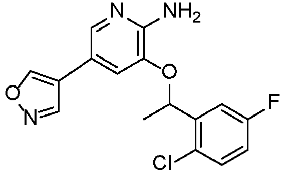

58 mg of dark brown solids were obtained with a yield of 24.04%. ¹H-NMR (400 MHz, DMSO): *δ* 8.86 (s, 1H), 7.51 (dd, *J* = 8.4 Hz, *J* = 5.2 Hz, 1H), 7.49 (dd, *J =* 9.2 Hz, *J =* 3.2 Hz, 1H), 7.37 (d, *J* = 1.6 Hz, 1H), 7.19 (td, *J* = 8.4 Hz, *J* = 3.2 Hz, 1H), 7.11 (t*, J* = 10.8 Hz, 1H), 6.74 (d, *J* = 1.6 Hz, 1H), 5.72 (m, 1H), 1.59 (d, *J=* 6.4 Hz, 3H). LC-MS: m/z: 334.1 (M+H)⁺.

### Example 47 Preparation of compound XFL-141

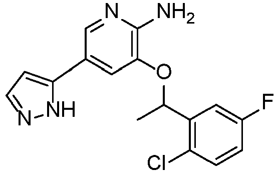

47 mg of light brown solids were obtained with a yield of 16.16%. ¹H-NMR (400 MHz, DMSO): *δ* 12.69 (s, 1H), 7.95 (s, 1H), 7.68 (d*, J* = 1.2 Hz, 1H), 7.53 (dd, *J =* 8.8 Hz, *J =* 5.2 Hz, 1H), 7.20 (s, 1H), 7.18 (td, *J =* 8.4 Hz, *J =* 3.2 Hz, 1H), 6.46 (s, 1H), 6.10 (s, 3H), 6.00 (s,1H), 5.78 (m, 1H), 1.62 (d, *J =* 6.0 Hz, 3H). LC-MS: m/z: 333.1 (M+H)⁺.

### Example 48 Preparation of compound XFL-147

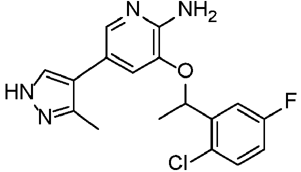

101 mg of yellow solids were obtained with a yield of 33.56%. ¹H-NMR (400 MHz, DMSO): *δ* 12.52 (s, 1H), 7.58 (s, 1H), 7.53 (dd, *J*= 8.8 Hz, *J*= 5.2 Hz, 1H), 7.47 (dd, *J =* 9.6 Hz, *J =* 3.2 Hz, 1H), 7.19 (td, *J=* 8.0 Hz, *J=* 3.2 Hz, 1H), 6.74 (d, *J* = 1.6 Hz, 1H), 5.85 (s, 1H), 5.76 (m, 1H), 2.11 (s, 3H), 1.62 (d, *J =* 6.4 Hz, 3H). LC-MS: m/z: 347.1 (M+H)⁺.

### Example 49 Preparation of compound XFL-169

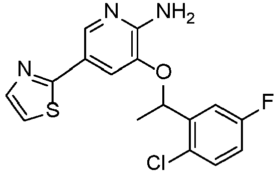

54 mg of yellow solids were obtained with a yield of 31.85%. ¹H NMR (400 MHz, CDCl₃) δ 8.12 (d*, J* = 1.8 Hz, 1H), 7.69 (d, *J =* 3.3 Hz, 1H), 7.31 (dd, *J*₁ = 8.8, *J₂* = 5.0 Hz, 1H), 7.23 (d, *J* = 1.7 Hz, 1H), 7.15 (d, *J =* 3.3 Hz, 1H), 7.08 (dd, *J*= 9.0, 3.0 Hz, 1H), 6.92 - 6.85 (m, 1H), 5.73 (q, *J* = 5.9 Hz, 1H), 5.39 (s, 2H), 1.63 (d, *J* = 6.3 Hz, 3H). LC-MS: m/z: 350.1 (M+H)⁺.

### Example 50 Preparation of compound XFL-171

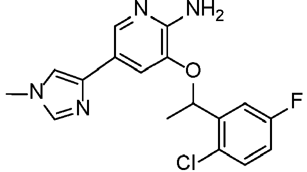

40 mg of light brown solids were obtained with a yield of 35.40%. ¹H NMR (400 MHz, CDCl₃) δ 7.94 (s, 1H), 7.33 (s, 1H), 7.26 (dd, *J*₁= 8.8, *J*₂ = 5.0 Hz, 1H), 7.11 (d*, J* = 3.0 Hz, 1H), 7.05 (d, *J* = 1.4 Hz, 1H), 6.85 (ddd, *J*₁ = 8.7, *J*₂ = 7.7, *J*₃ = 3.1 Hz, 2H), 5.68 (q, *J* = 6.3 Hz, 1H), 4.85 (s, 2H), 3.60 (s, 3H), 1.59 (d, *J=* 6.3 Hz, 3H). LC-MS: m/z: 347.1 (M+H)⁺.

### Example 51 Preparation of compound XFL-179

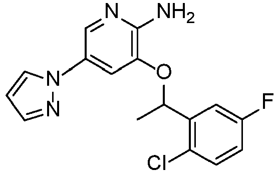

20 mg of yellow solids were obtained with a yield of 10.36%. ¹H NMR (400 MHz, CDCl₃) δ 7.90 (s, 1H), 7.59 (s, 2H), 7.29 (dd, *J*₁ = 8.8, *J*₂ = 5.0 Hz, 1H), 7.10 (d, *J=* 3.0 Hz, 1H), 7.04 (s, 1H), 6.88 (ddd, *J*₁ = 8.7, *J*₂ = 7.7, *J*₃ = 3.0 Hz, 1H), 6.33 (t*, J* = 2.1 Hz, 1H), 5.68 (q, *J* = 6.3 Hz, 1H), 4.87 (s, 2H), 1.62 (d, *J* = 6.3 Hz, 3H). LC-MS: m/z: 333.1 (M+H)⁺.

### Example 52 Preparation of compound XFL-201

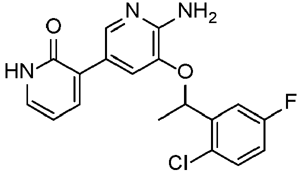

40 mg of dark brown solids were obtained with a yield of 35.40%. ¹H NMR (600 MHz, DMSO) δ 11.67 (s, 1H), 7.92 (s, 1H), 7.49 (ddd, *J*₁ = 13.7, *J*₂ = 9.0, *J*₃ = 2.1 Hz, 3H), 7.34 (s, 1H), 7.27 (d, *J=* 5.0 Hz, 1H), 7.19 - 7.14 (m, 1H), 6.21 (t, *J=* 6.6 Hz, 1H), 6.03 (s, 2H), 5.72 (q*, J* = 6.0 Hz, 1H), 1.58 (d, *J* = 6.3 Hz, 3H). LC-MS: m/z: 360.1 (M+H)⁺.

### Example 53 Preparation of compound XFL-1146-13

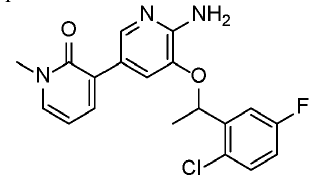

40 mg of black solids were obtained with a yield of 35.40%. ¹H NMR (400 MHz, DMSO) δ 7.94 (s, 1H), 7.63 (dd, *J*₁ = 6.7, *J*₂ = 1.9 Hz, 1H), 7.55 (dd, *J*₁ = 9.6, *J*₂ =3.1 Hz, 1H), 7.49 (dd, *J*₁ = 8.9, *J*₂ =5.1 Hz, 1H), 7.45 (dd, *J*₁ = 7.0, *J*₂ =2.0 Hz, 1H), 7.32 (d, *J* = 1.6 Hz, 1H), 7.17 (td, *J*₁ = 8.5, *J*₂ = 3.1 Hz, 1H), 6.24 (t, *J=* 6.8 Hz, 1H), 6.12 (s, 2H), 5.75 (q, *J=* 6.1 Hz, 1H), 3.45 (s, 3H), 1.58 (d, *J* = 6.3 Hz, 3H). LC-MS: m/z: 374.1 (M+H)⁺.

### Example 54 Preparation of compound XFL-1146-23

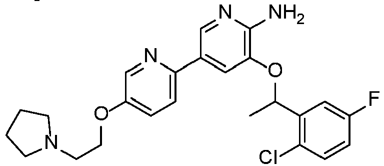

66 mg of brown oily compound wase obtained with a yield of 34.38%. ¹H NMR (400 MHz, DMSO) δ 8.28 (d, *J =* 2.9 Hz, 1H), 8.16 (d, *J* = 1.5 Hz, 1H), 7.68 (d, *J* = 8.8 Hz, 1H), 7.56 - 7.53 (m, 1H), 7.53 - 7.49 (m, 1H), 7.47 (d, *J*= 1.5 Hz, 1H), 7.42 (dd, *J*₁ = 8.8, *J*₂ = 2.9 Hz, 1H), 7.17 (td, *J*₁ = 8.5, *J*₂ = 3.1 Hz, 1H), 6.19 (s, 2H), 5.83 (q, *J=* 6.1 Hz, 1H), 4.28 (t, *J=* 5.2 Hz, 2H), 3.16 (s, 2H), 2.89 (s, 4H), 1.80 (s, 4H), 1.60 (d, *J=* 6.3 Hz, 3H). HRMS (EI) (m/z): [M]+ calculated for 456.1728; found 456.1729.

### Example 55 Preparation of compound XFL-1146-29

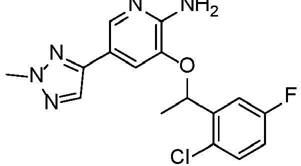

46 mg of light brown solids were obtained with a yield of 31.57%. ¹H NMR (400 MHz, CDCl₃) δ 7.97 (d, *J =* 1.4 Hz, 1H), 7.53 (s, 1H), 7.30 (dd, *J*₁ = 8.8, *J*₂ =5.0 Hz, 1H), 7.09 (dd, *J*₁ = 9.1, *J*₂ =3.0 Hz, 1H), 7.00 (d, *J =* 1.5 Hz, 1H), 6.90 - 6.84 (m, 1H), 5.69 (q, *J = 6.3* Hz, 1H), 4.98 (s, 2H), 4.11 (s, 3H), 1.62 (d, *J =* 6.3 Hz, 3H). LC-MS: m/z: 348.1(M+H)⁺.

### Example 56 Preparation of compound XFL-1146-31

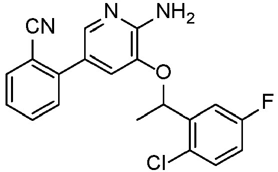

120 mg of yellow solids were obtained with a yield of 44.98%. ¹H NMR (400 MHz, CDCl₃) δ 7.75 (s, 1H), 7.65 - 7.61 (m, 1H), 7.51 (td, *J =* 7.7, 1.3 Hz, 1H), 7.31 (dd, *J*= 7.8, 6.8 Hz, 2H), 7.26 (d, *J* = 5.2 Hz, 1H), 7.11 (dd, *J* = 9.1, 3.0 Hz, 1H), 6.90 - 6.86 (m, 1H), 6.85 (d, *J* = 1.7 Hz, 1H), 5.66 (q, *J= 6.2* Hz, 1H), 4.96 (s, 1H), 1.63 (d, *J* = 6.3 Hz, 3H). LC-MS: m/z: 368.1(M+H)⁺.

### Experimental example. Test of activities of JAK2 inhibitor at molecular level

### Experimental principle:

JAK2 can catalyze the transfer of a phosphate group of adenosine triphosphate (ATP) to a polypeptide substrate, and the polypeptide substrate is labeled with two fluorescent groups, coumarin and fluorescein. Based on the method of fluorescence resonance energy transfer (FRET), JAK2 catalyzes the reaction of ATP to cause the two fluorophores to approach, the donor (coumarin) is excited at 400 nM, part of the energy is released, and the emission wavelength is 445 nM, while the other part of energy is transferred to fluorescein, and the emission wavelength is 520 nM. Different compounds have different degrees of inhibition on JAK2, leading to different degrees of phosphorylation of substrate. Therefore, the inhibition rates of different compounds can be calculated by measuring the ratio of enzyme-catalyzed substrate phosphorylation percentages.

### Experimental methods:

10 µL reaction system, 2.5 µL test compound, 5 µL kinase/peptide substrate mixture and 2.5 µL ATP solution were added into a 384-well plate, shaken for 30s, and incubated at room temperature for 1 hour. 5 µL of proteolytic enzyme and 15 µL of the reaction system were added and shaken for 30s and incubated at room temperature for 1 h. 5 µL of stop reagent was added, and the reaction system with a total volume of 20 µL was shaken for 30 seconds. A microplate reader was used to detect the fluorescence signal. The excitation wavelength is 400 nm and the emission wavelength is 445 nm and 520 nm, respectively. The inhibition rates of the compound at 7 concentration gradients were determined, and the IC₅₀ value for each compound was calculated by fitting curve with Origin 8.0. During the experiment, a positive control was set to confirm the feasibility of the reaction system, and each experiment was performed in triplicate. Ruxolitinib was used as a positive control during the experiment, and each experiment was performed at least in triplicate.

**Table 1 Results of JAK2 kinase activity test**

| | | | | | |
|---|---|---|---|---|---|
| 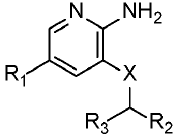 | | | | | |

| | R₁ | R₂ | R₃ | X | IC₅₀/µM |
|---|---|---|---|---|---|
| WWQ-131 | 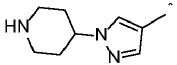 | 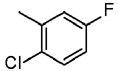 | CH₃ | O | 0.0067±0.001 |
| WWQ-133 | 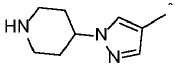 | 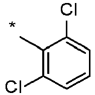 | CH₃ | O | 0.442±0.003 |
| WWQ-153 | 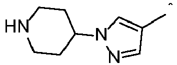 | 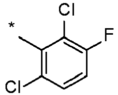 | H | O | 0.572±0.020 |
| WWQ-175 | 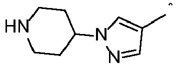 | 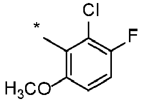 | CH₃ | O | 0.0265±0.0265 |
| WWQ-189 | 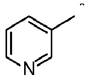 | 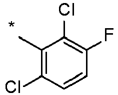 | CH₃ | O | 3.049±0.204 |
| WWQ-191 | 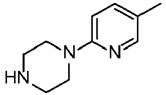 | 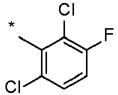 | CH₃ | O | 0.804±0.009 |
| WWQ-197 | 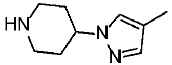 | 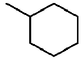 | CH₃ | O | 3.400±0.431 |
| WWQ-1093-13 | 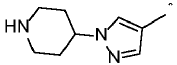 | 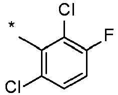 | C₂H₅ | O | 0.100±0.002 |
| WWQ-1093-23 | 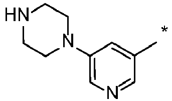 | 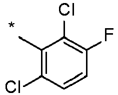 | CH₃ | O | 0.1191±0.0082 |
| WWQ-1093-25 | 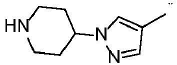 | 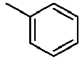 | CH₃ | O | 0.2152±0.0124 |
| WWQ-1093-27 | 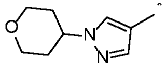 | 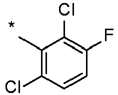 | CH₃ | O | 0.0647±0.0004 |
| WWQ-1093-65 | 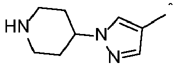 | 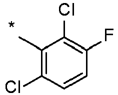 | CH₃ | O | 0.0917±0.0069 |
| WWQ-1093-71 | 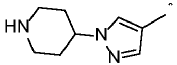 | 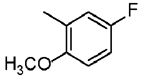 | CH₃ | O | 0.003±0.0001 |
| WWQ-1093-73 | 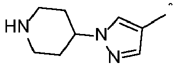 | 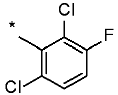 | CH₃ | NH | 0.1263±0.0053 |
| WWQ-1093-75 | 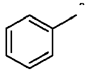 | 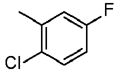 | CH₃ | O | 0.461±0.002 |
| WWQ-1093-81 | 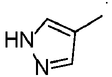 | 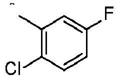 | CH₃ | O | 0.016±0.0001 |
| WWQ-1093-83 | 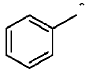 | 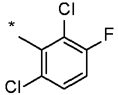 | CH₃ | O | / |
| WWQ-1093-85 | 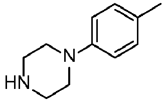 | 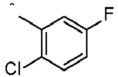 | CH₃ | O | 0.037±0.003 |
| WWQ-1093-87 | 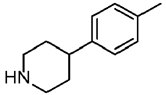 | 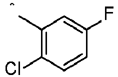 | CH₃ | O | 0.048±0.008 |
| WWQ-1093-89 | 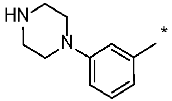 | 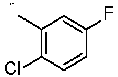 | CH₃ | O | 0.027±0.002 |
| WWQ-1093-91 | 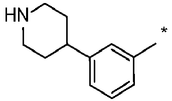 | 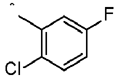 | CH₃ | O | 0.035±0.001 |
| **Ruxolitinib** | 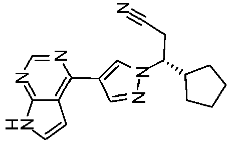 | | | | 0.592±0.044(nM) |
| **Crizotinib** | 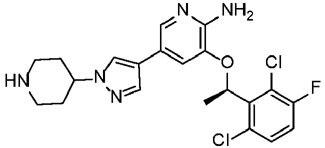 | | | | 16(nM) |

### Principle of CCK-8 cell proliferation detection:

WST-8 (chemical name: 2-(2-methoxy-4-nitrophenyl)-3-(4-nitrophenyl)-5-(2,4-disulfo benzene)-2H- tetrazolium monosodium), which is reduced to a highly water-soluble yellow formazan product (Formazan dye) by the dehydrogenase in cells under the action of the electron carrier dimethyl 1-methoxy-5-methylphenazinium sulfate (1-Methoxy PMS). The amount of the produced formazan is proportional to the number of living cells. Therefore, the survival rate of cells can be calculated indirectly by measuring the absorbance at 450 nm. The half inhibition rate of the compound on the proliferation of H3122, SET2, HEL cells can be determined by the CCK-8 method.

### Experimental method:

RPIM medium containing 10% FBS was used to culture cells (100 µL per well), and 3500 cells were inoculated into a 96-well plate, and placed in a 37°C, 5% CO₂ incubator for 24h, until 50%-70% of the cell confluence was achieved. Drugs with different concentration gradients were added respectively, and incubated for another 72hrs. 10µL of CCK-8 was added to each well, shaken, and incubated at 37°C for 2 h. And finally the absorption value of each well was measured at 450 nm. Finally, the IC₅₀ value was calculated by origin software, and the results are shown in Table 2.

### Principle of ALK kinase IC₅₀ test:

According to HTRF method, the peptide substrate was labeled with a biotin, which can specifically bind to XL665-labeled streptavidin. When the kinase adds a phosphate group to the polypeptide substrate in the first step, its Eu-labeled specific phosphorylation antibody can recognize and bind to it. In this way, in the second step of detection, a complex of XL665-labeled streptavidin, phosphorylated peptide substrate and Eu-labeled phosphorylation antibody can be formed. Since the distance between XL665 and Eu is short, FRET can be formed. On the other hand, Eu has a relatively long fluorescence half-life and can be applied to time-resolved fluorescence detection.

### Experimental method:

10 µL of reaction system, 4 µL test compound, 2 µL kinase, 2 µL biotin-labeled substrate and 2 µL ATP, was added into a 384-well plate, shaken for 30s, and incubated at room temperature for 1 hour. 5 µL of Eu-labeled phosphorylation site-specific antibody and 5 µL of XL665-labeled streptavidin were added. The reaction system was shaken for 30s, and incubated at room temperature for 1 hour. A microplate reader was used to detect the fluorescence signal. The inhibition rates of the compounds under 8 concentration gradients were determined, and the IC50 value of each compound was calculated by fitting curve with Origin 8.0. During the experiment, a positive control was set to confirm the feasibility of the reaction system. Each experiment was performed in triplicate. The results are shown in Table 2.

NOTE: HEL, SET-2, H3122 cells were purchased from ATCC (American type culture collection), Z'-LYTETM kinase assay platform was purchased from Invitrogen, HTRF KinEASETM-TK was purchased from Cisbio, and Cell Counting Kit-8 was purchased from Beyotime.

**Table 2**

| No. | JAK2 IC₅₀/nM | ALK IC₅₀/nM | SET 2 (JAK2 V617F) IC₅₀/µM | HEL (JAK2 V617F) IC₅₀/µM | H3122 IC₅₀/nM |
|---|---|---|---|---|---|
| Ruxolitinib | 0.592±0.044(nM) | - | 13.948 | 13.116 | 27.161±3.391 |
| Crizotinib | 0.016±0.002 | <1 (nM) | 0.470 | 7.304 | 0.842±0.056 |
| WWQ-13 1 | 0.0067±0.001 | 0.089 | 1.335 | 9.779 | 1.591±0.014 |
| WWQ-133 | 0.442±0.003 | 0.511 | 0.879 | 14.978 | >10 |
| WWQ-153 | 0.572±0.020 | 0.987 | 2.377 | 37.927 | >10 |
| WWQ-175 | 0.0265±0.0265 | 0.423 | 0.916 | 11.294 | 4.833±0.082 |
| WWQ-189 | 3.049±0.204 | >10 | 11.081 | 13.154 | 6.321±0.128 |
| WWQ-191 | 0.804±0.009 | 3.442 | 3.224 | 30.133 | 1.165±0.093 |
| WWQ-197 | 3.400±0.431 | >10 | 7.022 | 12.941 | 20.698±0.394 |
| WWQ-1093-13 | 0.100±0.002 | 3.564 | 7.864 | 37.349 | 3.537±0.034 |
| WWQ-1093-23 | 0.1191±0.0082 | 2.442 | 2.894 | 10.371 | 1.542±0.034 |
| WWQ-1093-25 | 0.2152±0.0124 | 5.434 | 4.611 | 13.698 | 3.518±0.102 |
| WWQ-1093-27 | 0.0647±0.0004 | 0.855 | 1.676 | 8.049 | 7.178±0.838 |
| WWQ-1093-65 | 0.0917±0.0069 | 0.119 | 1.490 | 11.651 | 0.648±0.044 |
| WWQ-1093-71 | 0.003±0.0001 | 1.334 | 0.616 | 9.966 | <0.5 |
| WWQ-1093-73 | 0.1263±0.0053 | 3.447 | 2.199 | 13.371 | 4.366±0.005 |
| WWQ-1093-75 | 0.461±0.002 | >10 | 33.151 | 24.492 | 14.407±4.209 |
| WWQ-1093-81 | 0.016±0.0001 | 0.334 | 5.553 | 4.696 | 2.551±0.160 |
| WWQ-1093-83 | >10 | >10 | 11.599 | 11.341 | 6.059±0.081 |
| WWQ-1093-85 | 0.037±0.003 | 0,498 | 8.262 | 35.753 | 1.135±0.012 |
| WWQ-1093-87 | 0.048±0.008 | 0.545 | 9.700 | 35.435 | >10 |
| WWQ-1093-89 | 0.027±0.002 | 0.334 | 2.259 | 8.426 | <0.5 |
| WWQ-1093-91 | 0.035±0.001 | 1.254 | 2.360 | 8.770 | 0.956 |
| MXY-89 | 0.695±0.002 | 7.889 | 11.018 | 2.443 | 6.874±0.022 |
| MXY-97 | 0.061±0.007 | 1.234 | 4.508 | 1.853 | 3.300±0.050 |
| MXY-111 | 0.006±0.349 | 0.132 | 2.484 | 12.431 | 0.988±0.047 |
| MXY-137 | 0.925±0.102 | 5.498 | 12.486 | 1.665 | 50.591±8.007 |
| MXY-141 | >10. | >10 | 13.209 | 12.209 | >100 |
| MXY-171 | 3.355±0.063 | >10 | 2.140 | 2.114 | 17.566±0.161 |
| MXY-175 | 1.101±0.081 | >10 | 10.992 | 2.231 | >100 |
| MXY-181 | 0.145±0.010 | 2.336 | >50 | 48.980 | 62.522±4.782 |
| MXY-193 | 0.008±0.004 | >10 | >50 | 38.970 | 78.753±2.274 |
| MXY-191 | 2.676±0.050 | >10 | >50 | 23.130 | >100 |
| MXY -032-45 | 0.566±0.029 | 3.674 | >10 | >10 | >100 |
| MXY -032-37 | 9.963±0.035 | >10 | >50 | >50 | >100 |
| MXY-032-51 | 0.025±0.001 | 0.335 | 31.210 | 4.665 | 9.910±1.277 |
| MXY -032-53 | 0.027±0.001 | 1.242 | 44.050 | 4.545 | 6.336±1.905 |
| MXY -032-55 | 0.528±0.008 | 3.886 | 18.640 | 5.989 | 11.573±6.021 |
| MXY -032-37 | 0.479±0.003 | 5.687 | >10 | 8.764 | 20.788±5.870 |
| MXY -032-69 | >5 | >10 | >50 | 10.680 | 25.262±6.242 |
| MXY -032-85 | >10 | >10 | >50 | 5.735 | 12.002±2.323 |
| MXY -032-87 | >10 | >10 | >50 | 9.473 | 17.403±0.250 |
| MXY-032-91 | 0.157±0.031 | 5.281 | 6.343 | 4.556 | 2.077±0.803 |
| MXY -032-93 | 0.015±0.001 | 2.334 | 2.877 | 3.453 | 1.975±0.840 |
| MXY -032-95 | 0.011±0.001 | 2.356 | 3.898 | 2.344 | 1.359±0.869 |
| XFL-107 | 0.844±0.100 | 3.374 | 3.348 | 9.136 | 1.448±0.118 |
| XFL-111 | 0.024±0.002 | 0.201 | 7.560 | 9.055 | 3.525±0.605 |
| XFL-137 | >10 | >10 | >10 | >50 | >100 |
| XFL-141 | 0.3021±0.003 | 3..373 | 2.483 | 5.411 | 78.617±3.003 |
| XFL-147 | 0.0408±0.0006 | 0.438 | 4.055 | 6.153 | 2.100±0.344 |
| XFL-169 | 1.117±0.014 | >10 | 8.167 | 15.910 | >100 |
| XFL-171 | 1.844±0.025 | >10 | 4.467 | 13.500 | 11.94±0.31 |
| XFL-179 | 0.931±0.032 | 7.776 | >50 | >50 | >100 |

Since the compound MXY-193 exhibits a very high selectivity while maintaining its activity at a single nanomolar level, it was subjected to chiral column resolution. The resolution method is described as follows:

| Column | Superchiral S-OD |
|---|---|
| Column size | 0.46 cm I.D. × 15 cm, 5um |
| Inj ection | 3 ul |
| Mobile phase | MeOH / DEA = 100/0.05 (v/v/v) |
| Flow rate | 0.8 ml/min |
| Wavelength | UV 220, 254 nm |
| Temperature | 40.0 °C |
| Rotating detector | Advanced laser polarizer |
| Sample structure | MXY-193 , raw material |

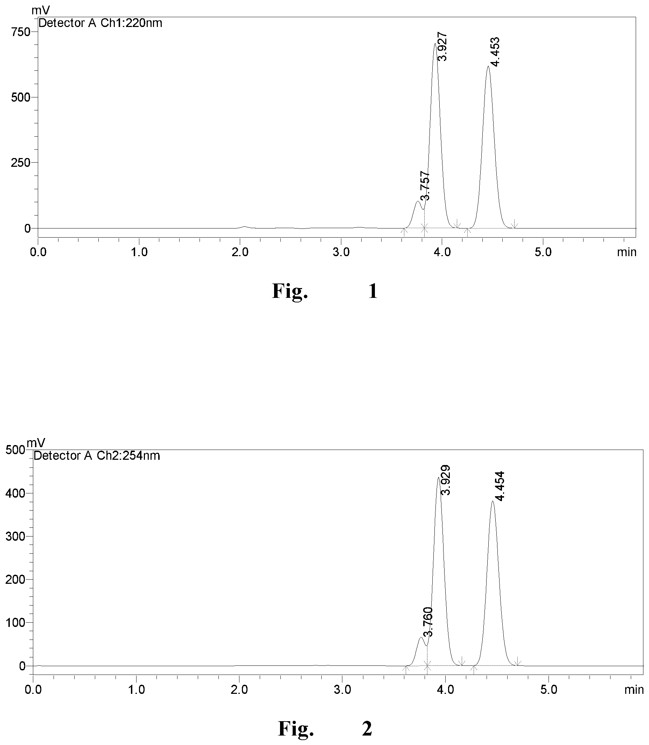

The resolution results are shown in Tables 3A, 3B, Figures 1 and 2;

**Table 3A Chromatographic peak table: 220 nm**

| No. | t_{R}(min) | Area | Area% | T. plate | Tailing | Degree of separation |
|---|---|---|---|---|---|---|
| 1 | 3.757 | 676097 | 6.2813 | 2910.445 | -- | -- |
| 2 | 3.927 | 5026115 | 46.6951 | 6046.936 | -- | 0.709 |
| 3 | 4.453 | 5061483 | 47.0237 | 6180.037 | 1.100 | 2.454 |

**Table 3A Chromatographic peak table: 254 nm**

| No. | t_{R}(min) | Area | Area% | T. plate | Tailing | Degree of separation |
|---|---|---|---|---|---|---|
| 1 | 3.760 | 416648 | 6.2690 | 2999.444 | -- | -- |
| 2 | 3.929 | 3116954 | 46.8988 | 6085.704 | -- | 0.709 |
| 3 | 4.454 | 3112526 | 46.8322 | 6222.500 | 1.104 | 2.461 |

The activities of the two configurations: configuration 1 (peak 1, R configuration) and configuration 2 (peak 2, S configuration) are shown in Table 4:

**Table 4**

| Configuration | JAK2 IC₅₀/µM | ALK IC₅₀/µM | SET 2 (JAK2 V617F) IC₅₀/µM | HEL (JAK2 V617F) IC₅₀/µM | H3122 IC₅₀/µM |
|---|---|---|---|---|---|
| 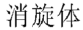 | 0.008±0.004 | >10 | >50 | 38.970 | 78.753±2.274 |
| Configuration 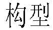 | 0.001±0.002 | 6.5 | 20.654 | 15.435 | 56.665 |
| Configuration 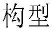 2 | 0.016±0.001 | >10 | >50 | >50 | >100 |

All documents mentioned in the present invention are cited as references in this application, as if each document is individually cited as a reference. In addition, it should be understood that after reading the above teachings of the present invention, a skilled person in the art can make various changes or modifications to the present invention, and these equivalent forms shall also fall within the scope defined by the appended claims of the present application.

## Claims

1. A compound of formula I or a stereoisomer or optical isomer, or a pharmaceutically acceptable salt, prodrug or solvate thereof, 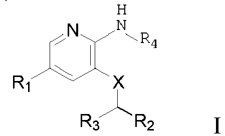 wherein
R₁ is selected from the following group: a hydrogen, halogen, C₁-C₁₀ alkyl, halogenated C₁-C₁₀ alkyl, C₁-C₁₀ alkoxy, halogenated C₁-C₁₀ alkoxy, substituted or unsubstituted phenyl, substituted or unsubstituted benzyl, substituted or unsubstituted 5-membered or 6-membered heterocyclic ring, substituted or unsubstituted 9-membered or 10-membered heterocyclic ring, -NR₅(R₆); the "substituted" means that the above group is replaced by oxo(=O) or one or more of the hydrogen atoms on the above group are replaced by a group selected from the following group: a C₁-C₄ alkyl (preferably methyl), cyano, -R₇-O-R₈, substituted or unsubstituted 4- to 6-membered ring containing one or two heteroatoms selected from nitrogen, oxygen or sulfur;
R₅ is selected from the following group: a hydrogen, C₁-C₄ alkyl, halogenated C₁-C₄ alkyl; R₆ is selected from the following group: C₁-C₁₀ alkyl, halogenated C₁-C₁₀ alkyl, substituted or unsubstituted phenyl, substituted or unsubstituted benzyl, substituted or unsubstituted 5-membered or 6-membered heterocycle, a substituted or unsubstituted 9-membered or 10-membered heterocycle; wherein the "substituted" means that one or more hydrogen atoms of the above group are replaced by a group selected from the following group: C₁-C₄ alkyl, cyano, halogen;
R₇ is an unsubstituted or substituted or unsubstituted C₁-C₆ alkylene group; R₈ is selected from the following group: a C₁-C₆ alkyl, halogenated C₁-C₆ alkyl, substituted or unsubstituted 4- to 6-membered cyclic group containing one or two heteroatoms selected from nitrogen, oxygen or sulfur, substituted or unsubstituted 4- to 6-membered cyclic group-C₁-C₄ alkyl containing one or two heteroatoms selected from nitrogen, oxygen or sulfur;
R₂ is selected from the following group: a substituted or unsubstituted phenyl, substituted or unsubstituted pyridyl, substituted or unsubstituted pyrimidine or substituted or unsubstituted C₅-C₇ cycloalkyl; and the "substituted" means that one or more hydrogen atoms of the above group is replaced by a group selected from the following group: a hydrogen, halogen, C₁-C₃ alkyl, halogenated C₁-C₃ alkyl, C₁-C₃ alkoxy, halogenated C₁-C₃ alkoxy;
R₃ is selected from the following group: a hydrogen, halogen, C₁-C₁₀ alkyl, halogenated C₁-C₁₀ alkyl, hydroxy, C₁-C₁₀ alkoxy;
R₄ is selected from the group consisting of a hydrogen, C₁-C₁₀ alkyl, C₁-C₁₀ acyl;
X is selected from the following group: CH₂, O, NH, S, SO, SO₂;
Unless otherwise specified, the "substituted" as mentioned above means that one or more hydrogens of a group are replaced by a group selected from the following group: a C₁-C₄ alkyl, C₁-C₄ haloalkyl and halogen.

2. The compound of claim 1 or a stereoisomer or optical isomer, or a pharmaceutically acceptable salt, prodrug or solvate thereof, wherein the compound is represented by formula II: 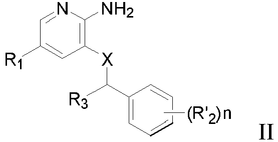 wherein
the definitions on R₁ and R₃ are the same as above;
R₂' is selected from the group consisting of a hydrogen, halogen, C₁-C₃ alkyl, halogenated C₁-C₃ alkyl, C₁-C₃ alkoxy, and halogenated C₁-C₃ alkoxy;
X is selected from the following group: O, NH, S;
n is 1, 2, 3, or 4.

3. The compound of claim 1 or 2 or a stereoisomer or optical isomer, or a pharmaceutically acceptable salt, prodrug or solvate thereof, wherein the 5-membered or 6-membered heterocyclic ring or the 9-membered or 10-membered heterocyclic ring contains at least one nitrogen heteroatom.

4. The compound of claim 1 or 2 or a stereoisomer or optical isomer, or a pharmaceutically acceptable salt, prodrug or solvate thereof, wherein the 5-membered or 6-membered heterocyclic ring is selected from the following group: pyrrole, pyrazole, pyridine.

5. The compound of claim 1 or 2 or a stereoisomer or optical isomer, or a pharmaceutically acceptable salt, prodrug or solvate thereof, wherein the 9-membered or 10-membered heterocyclic ring is selected from the following group:

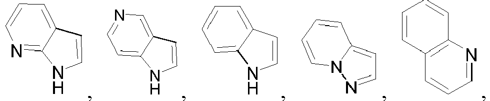

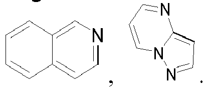

6. The compound of claim 1 or 2 or a stereoisomer or optical isomer, or a pharmaceutically acceptable salt, prodrug or solvate thereof, wherein,
R₁ is selected from the following group: a substituted or unsubstituted 5-membered or 6-membered heterocyclic ring; wherein the "substituted" means that one or more hydrogen atoms of the above group are replaced by a group selected from the following group: a C₁-C₄ alkyl, cyano, -R₇-O-R₈, saturated 4- to 6- membered ring containing one or two heteroatoms selected from nitrogen, oxygen or sulfur; wherein R₇ is a C₁-C₆ alkylene; and R₈ is selected from the group consisting of a C₁-C₆ alkyl and halogenated C₁-C₆ alkyl; or
R₁ is selected from the following group:

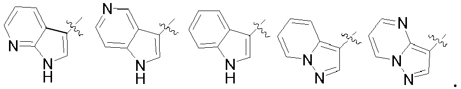

7. The compound of claim 1 or 2 or a stereoisomer or optical isomer, or a pharmaceutically acceptable salt, prodrug or solvate thereof, wherein the 4- to 6- membered ring containing one or two heteroatoms selected from nitrogen, oxygen or sulfur is selected from the group consisting of piperidine, piperazine, morpholine, oxetane and tetrahydrofuran.

8. The compound of claim 2 or a stereoisomer or optical isomer, or a pharmaceutically acceptable salt prodrug or solvate thereof, wherein 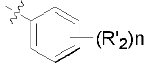 is a group selected from the following group: 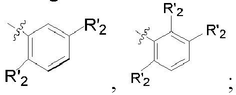 wherein the definitions on R₂' are the same as above.

9. The compound of claim 1 or a stereoisomer or optical isomer, or a pharmaceutically acceptable salt, prodrug or solvate thereof, wherein the compound is selected from the following group:

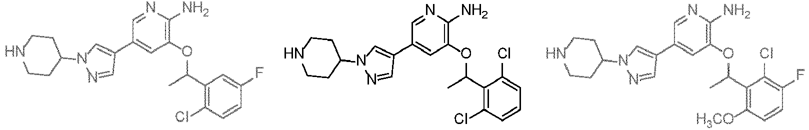

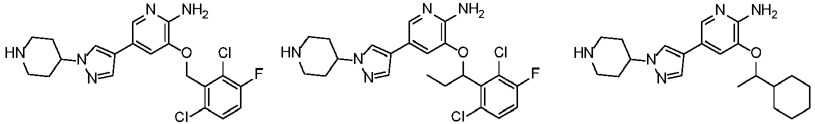

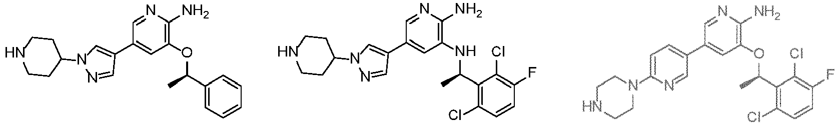

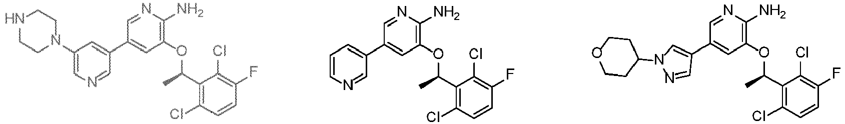

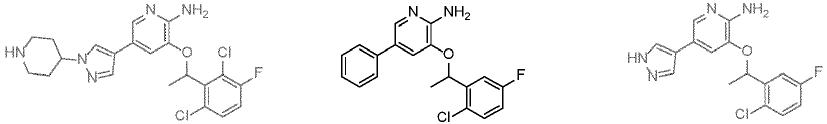

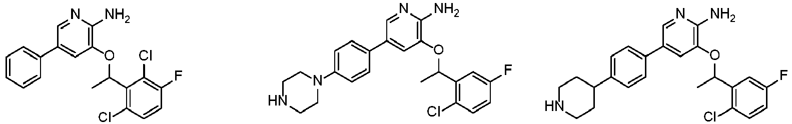

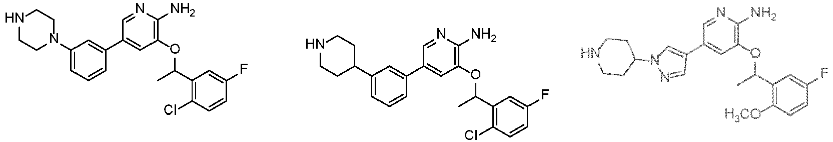

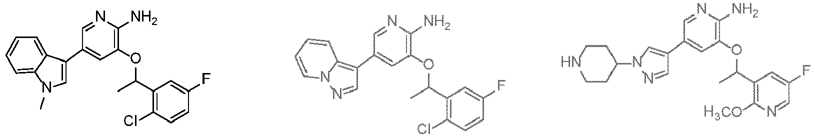

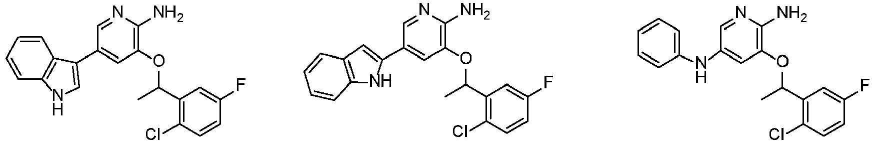

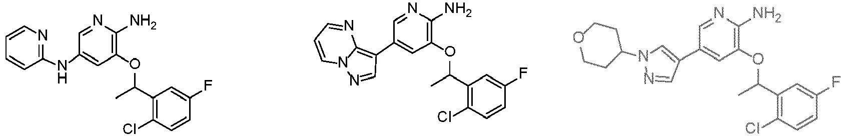

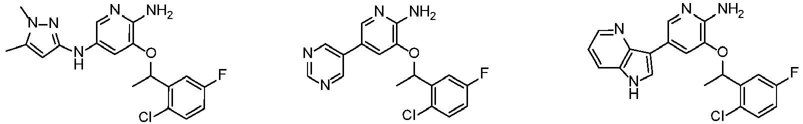

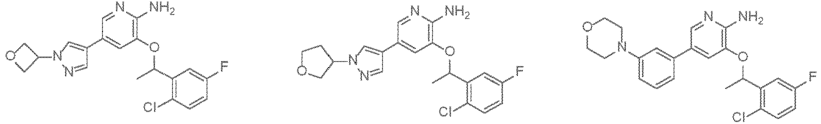

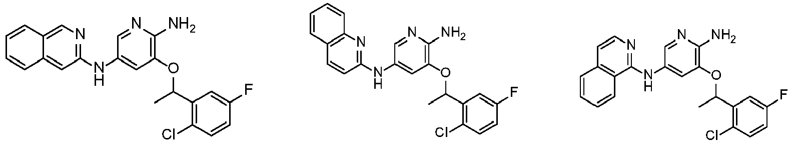

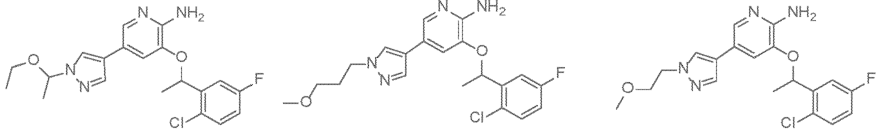

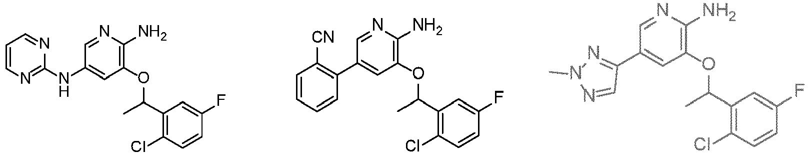

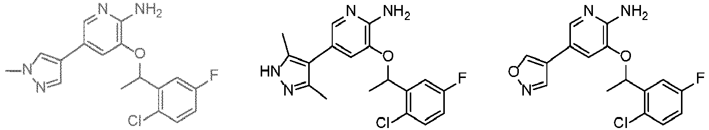

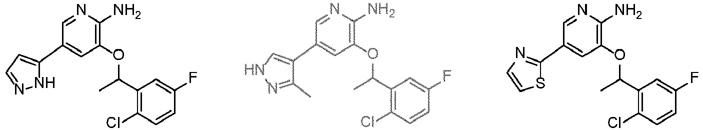

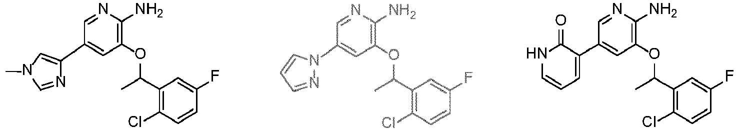

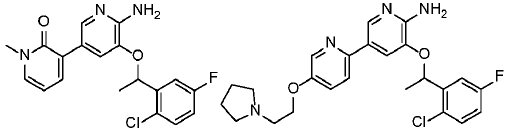

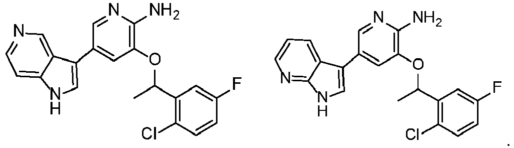

10. A pharmaceutical composition, comprising the compound of any one of claims 1-5 or a stereoisomer or optical isomer, or a pharmaceutically acceptable salt, prodrug or solvate thereof, and a pharmaceutically acceptable carrier or excipient.

11. Use of the compound of any one of claims 1-9 or a stereoisomer or optical isomer, or pharmaceutically acceptable salt, prodrug or solvate thereof, for preparing a medicament for preventing or treating JAK2-mediated diseases; and/or for preparing JAK2 inhibitors.

12. The use of claim 11, wherein the JAK2-mediated disease is myelodysplastic syndrome (MDS), eosinophilia, tumor, inflammatory disease, or infection caused by bacteria, viruses or fungi.

13. The use of claim 12, wherein,
the tumor is selected from the group consisting of: myeloproliferative carcinoma (MPN), melanoma, lung cancer (preferably non-small cell lung cancer), kidney cancer, ovarian cancer, prostate cancer, breast cancer, colon cancer, bone Cancer, pancreatic cancer, skin cancer, head and neck cancer, uterine cancer, rectal cancer, anal cancer, stomach cancer, testicular cancer, fallopian tube cancer, endometrial cancer, cervical cancer, vagina cancer, vulvar cancer, Hodgkin's disease, non Hodgkin's lymphoma, esophageal cancer, small bowel cancer, endocrine system cancer, thyroid cancer, parathyroid cancer, adrenal cancer, soft tissue sarcoma, urethral cancer, penile cancer, acute myeloid leukemia, chronic myelogenous leukemia, acute lymphoblastic leukemia , chronic lymphocytic leukemia, pediatric solid tumors, lymphocytic lymphoma, bladder cancer, kidney or ureter cancer, renal pelvis cancer, central nervous system (CNS) tumors, primary CNS lymphomas, tumor angiogenesis, spinal axons, glioma of brain stem, pituitary adenoma, Kaposi's sarcoma, epidermoid carcinoma, squamous cell carcinoma, T cell lymphoma; and / or
the inflammatory disease is selected from the group consisting of rheumatoid arthritis, ankylosing spondylitis, autoimmune hemolytic anemia, arthritis, myasthenia gravis, systemic lupus erythematosus, pernicious anemia, polymyositis; and / or
the virus is selected from the group consisting of hepatitis virus (type A, B and C), sporangia virus, influenza virus, adenovirus, coronavirus, measles virus, dengue fever virus, polio virus, rabies virus; and / or
the bacteria are selected from the group consisting of chlamydia, rickettsiae, mycobacteria, staphylococci, pneumococcus, vibrio cholerae, and clostridium tetani; and / or
the fungus is selected from the group consisting of Candida, Aspergillus, and Saccharomyces dermatitis.

14. A JAK2 inhibitor, comprising the compound of any one of claims 1-9 or a stereoisomer or optical isomer, or a pharmaceutically acceptable salt, prodrug or solvate thereof or the pharmaceutical composition of claim 10.
